Europäisches Patentamt

European Patent Office

Office européen des brevets

(19)

(11) Publication number: **0 352 227**
**A2**

# (12) EUROPEAN PATENT APPLICATION

(21) Application number: **89810521.8**

(22) Date of filing: **11.07.89**

(51) Int. Cl.⁵: **C 07 K 7/10**
A 61 K 37/64, C 12 N 15/15,
C 12 P 21/02, C 12 N 15/70,
C 12 N 1/21, C 12 N 15/81,
C 12 N 1/19

(30) Priority: **19.07.88 GB 8817161**

(43) Date of publication of application:
**24.01.90 Bulletin 90/04**

(84) Designated Contracting States:
**AT BE CH DE ES FR GB GR IT LI LU NL SE**

(71) Applicant: **CIBA-GEIGY AG**
**Klybeckstrasse 141**
**CH-4002 Basel (CH)**

**UCP GEN-PHARMA AG**
**Solothurnstrasse 24**
**CH-3422 Kirchberg (CH)**

(72) Inventor: **Stone, Stuart R., Dr.**
**Claragraben 116**
**CH-4057 Basle (CH)**

**Dennis, Stanley, Dr.**
**Maulbeerstrasse 37**
**CH-4058 Basle (CH)**

**Hofsteenge, Jan, Dr.**
**Mischelistrasse 15**
**CH-4153 Reinach (CH)**

**Meyhack, Bernd, Dr.**
**Höhenweg 9**
**CH-4312 Magden (CH)**

**Wallace, Andrew, Dr.**
**Klybeckstrasse 122**
**CH-4057 Basle (CH)**

**Grossenbacher, Hugo, Dr.**
**Martin-Distelistrasse 79**
**CH-4600 Olten (CH)**

The applicant has filed a statement in accordance with Rule 28 (4) EPC (issue of a sample only to an expert). Accession number(s) of the deposit(s): DSM 3665 and DSM 4084.
Claims for the following Contracting States: ES + GR.

(54) **Modified proteins.**

(57) Novel polypeptides are provided which differ from desulphatohirudin by simple or multiple mutations. The novel polypeptides have valuable pharmacological properties and can be prepared by recombinant DNA techniques.

**Fig. 2:** Plasmid map of plasmid pJDB207/GAPFL-HIR

EP 0 352 227 A2

**Description**

**Modified proteins**

The present invention relates to modified proteins, to methods for the preparation thereof, to pharmaceutical compositions containing such proteins and to their use for the inhibition of enzymes.

Hirudin, the anticoagulant principle that occurs naturally in the leech Hirudo medicinalis, is a polypeptide of 65 amino acids with three disulphide bridges. Long known since the first investigations on leech saliva in 1884 and the pioneering work of Markwardt [Naturwissenschaften 42, 537 (1955); Methods Enzymol. 19, 924 (1970)], it was not until recently that the hirudin structure was elucidated by Dodt et. al. [FEBS Lett. 165, 180 (1984)]. The most recent findings are that hirudin exists in several variants, described as hirudin variant 1 (HV1), hirudin variant 2 (HV2), hirudin PA and other analogs.

Hirudin is the strongest thrombin inhibitor known, while other enzymes of the coagulation cascade are not affected. In contrast to heparin which is the preferred anticoagulant in conventional anticoagulation therapy, hirudin exerts its action directly on thrombin and, unlike the former, does not act through antithrombin III. The interaction between hirudin and thrombin occurs with extremely high potency. This potency is many orders of magnitude greater than the binding of thrombin to fibrinogen, to platelets or to antithrombin III. Therefore, even low concentrations of hirudin are able to overcome all the interactions of thrombin and their biological consequences. Furthermore, hirudin has a low toxicity, is non antigenic and shows almost complete clearance via the kidneys in a biologically active form.

Recently, cDNAs and synthetic genes coding for hirudin or hirudin variants have been cloned and expressed in microbial hosts such as Escherichia coli and Saccharomyces cerevisiae (cf. European Patent Applications No. 158564 and 168342). Although the expression products lack the sulphate monoester group at Tyr[63] - and were therefore designated "desulphatohirudins" - they turned out to exhibit approximately the same biological activity as natural hirudin in which the tyrosine residue is present as sulphate monoester.

Hirudin has been administered to man by both intravenous and subcutaneous routes. By intravenous route the mean elimination half life was found to be 0.84 h and approximately 50 % of the administered dose was excreted intact in the urine. After subcutaneous administration inhibitory levels of hirudin were found in the plasma for at least 4 h. Hirudin is therefore considered to be fairly short acting with a duration of action similar to heparin. Considering this drawback there is a strong need for selective antithrombotically active polypeptides exhibiting a longer half life in vivo. Corresponding polypeptides allowing once daily treatment will have advantages over existing therapies in deep vein and arterial thrombosis. It is an object of the present invention to provide such antithrombotically active polypeptides.

There is also a strong need for hirudin compounds which exhibit reduced antithrombotic potency. Reduction of the antithrombotic potency results in a reduction of the ability of these hirudin compounds to prevent thrombin interacting with its highest affinity ligands, notably the platelet receptor. However, by careful selection of the antithrombotic potency of the hirudin it retains its ability to inhibit fibrinogen clotting, a low affinity property of thrombin. Such hirudin compounds have the advantage in that they have inhibitory effects on thrombosis, a fibrin dominated event, but have little effect on haemostasis, a platelet dominated effect. It is a further object of the present invention to provide such hirudin compounds with reduced antithrombotic potency.

Surprisingly, it was found that the increase in hydrophobicity of hirudins occurring when acidic amino acid residues (Asp, Glu) in the C-terminus are replaced by lipophilic amino acids and/or the C-terminus is otherwise modified, for example by the addition of protease resistant amino acid residues such as Pro, leads to reduced antithrombotic potency and/or to a longer half life in vivo.

Accordingly, the present invention provides novel mutants of desulphatohirudin having the amino acid sequence

2

EP 0 352 227 A2

H-Val-Val-Tyr-Thr-Asp-Cys-Thr-Glu-Ser-Gly-

-Gln-Asn-Leu-Cys-Leu-Cys-Glu-Gly-Ser-Asn-

-Val-Cys-Gly-Gln-Gly-Asn-Lys-Cys-Ile-Leu-

-Gly-Ser-Asp-Gly-Glu-Lys-Asn-Gln-Cys-Val-

-Thr-Gly-Glu-Gly-Thr-Pro-Lys-Pro-Gln-Ser-

-His-Asn-$Y_1$-Gly-Asp-Phe-$Y_2$-$Y_3$-Ile-Pro-

-$Y_4$-$Y_5$-$Y_6$-Leu-$Y_7$-OH

(I)

wherein $Y_1$ represents Asp or the radical of a neutral genetically encoded amino acid, $Y_2$ and $Y_3$ independently from each other represent Glu, Asn or the radical of a lipophilic genetically encoded amino acid, $Y_4$ and $Y_5$ independently from each other represent Glu or the radical of a neutral genetically encoded amino acid, $Y_6$ represents Tyr or the radical of an acidic genetically encoded amino acid and $Y_7$ represents Gln or the dipeptidyl radical GlnPro, with the exception of compounds of the formula I in which $Y_1$ is Asp, $Y_6$ is Tyr, $Y_7$ is Gln, $Y_2$ and $Y_3$ are each Glu and one of the radicals $Y_4$ and $Y_5$ is Gln and the other is Glu, and salts thereof.

The mutants according to the invention differ from native desulphatohirudin ($Y_1$ is Asp, $Y_2$, $Y_3$, $Y_4$ and $Y_5$ are each Glu, $Y_6$ is Tyr and $Y_7$ is Gln) with respect to 1 to 4, in particular 1 or 2, amino acid radicals.

Neutral genetically encoded amino acids are the following L-amino acids: Ala, Ser, Thr, Val, Leu, Ile, Asn, Gln, Met, Phe, Trp and Pro, furthermore the amino acid Gly.

Lipophilic genetically encoded amino acids are the following L-amino acids: Ala, Val, Leu, Ile, Phe and Gly.

Acidic genetically encoded amino acids are Asp and Glu.

In particular, the invention relates to mutants of desulphatohirudin of the formula I wherein $Y_1$ is Asp, Ala, Leu or Asn, $Y_2$ and $Y_3$ independently from each other represent Glu, Leu or Asn, $Y_4$ and $Y_5$ independently from each other represent Glu, Gln, Asn or Leu, $Y_6$ is Tyr, Asp or Glu and $Y_7$ is Gln or the dipeptidyl radical GlnPro, with the exception of compounds of the formula I in which $Y_1$ is Asp, $Y_6$ is Tyr, $Y_7$ is Gln, $Y_2$ and $Y_3$ are each Glu and one of the radicals $Y_4$ and $Y_5$ is Gln and the other is Glu, and salts thereof.

Example of such desulphatohirudin mutants are [Gln[61,62]]-desulphatohirudin, [Leu[61,62]]-desulphatohirudin, [Asn[61,62]]-desulphatohirudin, [Leu[57,58,61,62]]-desulphatohirudin, [Asn[57,58,61,62]]-desulphatohirudin, [Ala[53]]-desulphatohirudin, [Asp[63]]-desulphatohirudin, [Glu[63]]-desulphatohirudin and [Pro[66]]-desulphatohirudin.

The compounds of the invention can be in the free form but also in the form of their salts. As they contain free amino groups in several amino acid residues, the compounds of the invention can be in the form of acid addition salts. Suitable acid addition salts are in particular pharmacologically acceptable salts with conventional therapeutically acceptable acids. Representative inorganic acids are hydrohalic acids (such as hydrochloric acid), and also sulfuric acid, phosphoric acid and pyrophosphoric acid. Representative organic acids are in particular arenesulfonic acids (such as benzenesulfonic or p-toluenesulfonic acid), or lower alkanesulfonic acids (such as methanesulfonic acid), as well as carboxylic acids such as acetic acid, lactic acid, palmitic acid, stearic acid, malic acid, tartaric acid, ascorbic acid and citric acid. As, however, the compounds according to the invention also contain free carboxyl groups in several amino acid residues, which carboxyl groups impart acidic character to the entire peptide, they can also be in the form of salts with inorganic or organic bases, e.g. sodium, potassium. calcium or magnesium salts, or also ammonium salts derived from ammonia or a pharmacologically acceptable organic nitrogen-containing base. However, as they contain at the same time free carboxyl groups and free amino groups, they can also be in the form of inner salts. Pharmacologically acceptable salts are prefered.

The method for the preparation of the desulphatohirudin mutants according to the invention comprises culturing a microbial host strain which has been transformed with a hybrid vector comprising an expression cassette consisting of a promoter operably linked to a first DNA sequence encoding a signal peptide linked in the proper reading frame to a second DNA sequence coding for the desulphatohirudin mutant, and a DNA

3

sequence containing transcription termination signals, and isolating the desulphatohirudin mutant, and, if desired, converting an obtained polypeptide having free carboxy and/or amino groups into a salt or converting a salt obtained into the free compound.

Suitable microbial host strains include, for example, strains of Bacillus subtilis, Escherichia coli and Saccharomyces cerevisiae.

The transformed microbial host strains are cultured in a liquid medium containing assimilatable sources of carbon, nitrogen and inorganic salts, applying methods known in the art.

Various carbon sources are usable. Example of preferred carbon sources are assimilable carbohydrates, such as glucose, maltose, mannitol, fructose or lactose, or an acetate such as sodium acetate, which can be used either alone or in suitable mixtures. Suitable nitrogen sources include, for example, amino acids, such as casamino acids, peptides and proteins and their degradation products, such as tryptone, peptone or meat extracts, furthermore yeast extract, malt extract, corn steep liquor, as well as ammonium salts, such as ammonium chloride, sulphate or nitrate which can be used either alone or in suitable mixture. Inorganic salts which may be used include, for example, sulphates, chlorides, phosphates and carbonates of sodium, potassium, magnesium and calcium. Additionally, the nutrient medium may also contain growth promoting substances. Substances which promote growth include, for example, trace elements, such as iron, zinc, manganese and the like, or individual amino acids.

Microbial host cells transformed with hybrid vectors tend to lose the latter. Such cells have to be grown under selective conditions, i.e. conditions which require the expression of a hybrid vector-encoded gene for growth. Most selective markers currently in use and present in the hybrid vectors according to the invention (infra) are genes coding for enzymes of amino acid or purine biosynthesis. This makes it necessary to use synthetic minimal media deficient in the corresponding amino acid or purine base. However, genes conferring resistance to an appropriate biocide may be used as well [e.g. a gene conferring resistance to the amino-glycoside G418]. Host cells transformed with vectors containing antibiotic resistance genes are grown in complex media containing the corresponding antibiotic whereby faster growth rates and higher cell densities are reached.

Host cells containing hybrid vectors with a constitutive promoter express the desulphatohirudin mutant gene controlled by said promoter without induction. However, if the desulphatohirudin mutant gene is under the control of a regulated promoter the composition of the growth medium has to be adapted in order to obtain maximum levels of mRNA transcripts, i.e. when using the PHO5 promoter in yeast the growth medium must contain a low concentration of inorganic phosphate for derepression of this promoter.

The cultivation is carried out by employing conventional techniques. The culturing conditions, such as temperature, pH of the medium and fermentation time are selected in such a way that maximal levels of desulphatohirudin mutants are produced. A chosen yeast or E. coli strain is preferably grown under aerobic conditions in submerged culture with shaking or stirring at a temperature of about 25° to 35°C, preferably at about 28°C, at a pH value of from 4 to 7, for example at approximately pH 5, and for at least 12 hours to 3 days, preferably for such a period that satisfactory yields of desulphatohirudin mutants are obtained.

Irrespective of the host strain, promoter and signal peptide used, most of the produced desulphatohirudin mutant is secreted into the culture medium or the periplasmic space whereas only a minor part remains associated with the cell interior. The precise ratio (secreted compounds/cell associated compounds) depends on the fermentation conditions.

The desulphatohirudin mutants can be isolated by conventional means. For example, the first step consists usually in separating the cells from the culture fluid by means of centrifugation. The resulting supernatant can be enriched for secreted desulphatohirudin mutants by treatment with polyethyleneimine so as to remove most of the non-proteinaceous material, and precipitation of the proteins by saturating the solution with ammonium sulphate. Host proteins, if present, can also be precipitated by means of acidification with acetic acid (for example 0.1 %, pH 4-5). A further enrichment of the desulphatohirudin mutant can be achieved by extracting the acetic acid supernatant with n-butanol. Other purification steps include, for example, desalination, chromatographic processes, such as ion exchange chromatography, gel filtration chromatography, partition chromatography, HPLC, reversed phase HPLC and the like. The separation of the constituents of the protein mixture is also effected by dialysis, according to charge by means of gel electrophoresis or carrier-free electrophoresis, according to molecular size by means of a suitable Sephadex column, by affinity chromatography, for example with antibodies, especially monoclonal antibodies, or with thrombin coupled to a suitable carrier for affinity chromatography, or by other processes, especially those known from the literature.

If it is desired to isolate desulphatohirudin mutants which have accumulated in the periplasmic space, some supplementary purification steps are required: The desulphatohirudin mutants are recovered by enzymatic removal of the cell wall (infra) or by treatment with chemical agents, e.g. thiol reagents or EDTA, or by subjecting the cell wall to osmotic shocks, which give rise to cell wall damages permitting the product to be released.

The test with anti-hirudin or anti-desulphatohirudin antibodies (for example, monoclonal antibodies obtainable from hybridoma cells), the thrombin test [M.U. Bergmeyer (ed.), Methods in Enzymatic Analysis, Vol. II, p. 314-316, Verlag Chemie, Weinheim (FRG) 1983] or the blood coagulation test [F. Markwardt et al., Thromb. Haemost. 47, 226 (1982)] can be used to detect the desulphatohirudin mutant activity in fractions obtained in the course of the purification procedure.

Depending on the method employed, the compounds of the invention are obtained in the free form or in the form of acid addition salts, inner salts or salts with bases. The free compound can be obtained in known manner from the acid addition salts or salts with bases. In turn, therapeutically acceptable acid addition salts can be obtained from the free compounds by reaction with acids, e.g. with those acids which form the above-mentioned salts, and by evaporation or lyophilisation. The inner salts can be obtained by adjusting the pH to a suitable neutral point.

The transformed microbial host cells according to the invention can be prepared by recombinant DNA techniques comprising the steps of
- preparing a hybrid vector comprising an expression cassette consisting of a promoter operably linked to a first DNA sequence encoding a signal peptide linked in the proper reading frame to a second DNA sequence coding for the desulphatohirudin mutant, and a DNA sequence containing transcription termination signals,
- transforming a microbial host strain with said hybrid vector,
- and selecting transformed microbial host cells from untransformed host cells.

Expression vectors

The hybrid vectors according to the invention comprise an expression cassette consisting of a promoter operably linked to a first DNA sequence encoding a signal peptide linked in the proper reading frame to a second DNA sequence coding for the desulphatohirudin mutant, and a DNA sequence containing transcription termination signals.

The selection of a suitable vector is determined by the microbial host cell provided for the transformation. Suitable hosts are those specified above, especially strains of Saccharomyces cerevisiae, and bacterial strains, especially strains of Escherichia coli and Bacillus subtilis.

Examples of vectors that are suitable for the expression of the desulphatohirudin mutant gene in an E. coli strain are bacteriophages, for example derivatives of the bacteriophage λ, or plasmids, such as the plasmid colE1 and its derivatives, for example pMB9, pSF2124, pBR317 or pBR322. Suitable vectors contain a complete replicon and a marker gene, which renders possible the selection and identification of the microorganisms transformed by the expression plasmids by means of a phenotype feature. Suitable marker genes impart to the microorganism, for example, resistance to heavy metals, antibiotics such as ampicillin or tetracyclin, and the like.

Several promoters can be used for regulating the expression cassette in E. coli. Especially promoters of strongly expressed genes are used. Suitable promoters are the lac, tac, trp and lpp promoters, furthermore the phage λN or the phage λpL promoter, and others. In the present invention, the preferred promoter for use in E. coli is the lpp and the lac promoter.

Vectors suitable for replication and expression in S. cerevisiae contian a yeast replication origin and a selective genetic marker for yeast. Hybrid vectors that contain a yeast replication origin, for example the chromosomal autonomously replicating segment (ars), are retained extrachromosomally within the yeast cell after transformation and are replicated autonomously during mitosis. Also, hybrid vectors that contain sequences homologous to the yeast 2μ plasmid DNA can be used. Such hybrid vectors are integrated by recombination in 2μ plasmids already present within the cell, or replicate autonomously. Suitable marker genes for yeast are especially those that impart antibiotic resistance to the host or, in the case of auxotrophic yeast mutants, genes that complement the host lesions. Corresponding genes impart, for example, resistance to the antibiotic cycloheximide or provide for prototrophy in an auxotrophic yeast mutant, for example the URA3, LEU2, HIS3 or the TRPI gene.

Preferably, yeast hybrid vectors furthermore contain a replication origin and a marker gene for a bacterial host, especially E. coli, so that the construction and the cloning of the hybrid vectors and their precursors can be carried out in E. coli. Promoters suitable for expression in yeast are, for example, those of the ADHI, ADHII, or PHO5 gene, and also promoters involved in glycolysis, for example the PGK promoter.

The DNA sequence encoding a signal peptide ("signal sequence") is derived from a gene of the microbial host coding for a polypeptide which is ordinarily secreted. When E. coli is used as the host microorganism the ompA, lpp, maltose binding protein, λ receptor, leucine binding protein or β-lactamase signal sequence may be chosen. For use in yeast the hirudin signal sequence obtainable from leech genome DNA can be chosen. More preferred signal sequences for use in yeast are, for example, the signal and prepro sequences of the yeast invertase, α-factor, pheromone peptidase (KEX1), "killer toxin" and repressible acid phosphatase (PHO5) genes and the glucoamylase signal sequence from Aspergillus awamori. Alternatively, fused signal sequences may be constructed by ligating part of the signal sequence (if present) of the gene naturally linked to the promoter used (for example PHO5), with part of the hirudin signal sequence. Those combinations are favoured which allow a precise cleavage between the signal sequence and the desulphatohirudin mutant amino acid sequence. Additional sequences, such as pro- or spacer-sequences which may or may not carry specific processing signals can also be included in the constructions to facilitate accurate processing of precursor molecules.

The DNA sequence containing transcription termination signals is preferably the 3′ flanking sequence of a gene derived from the selected microbial host which contains proper signals for transcription termination. Suitable 3′ flanking sequences are, for example, those of the gene naturally linked to the promoter used.

The hybrid vectors according to the invention can be prepared by methods known in the art, for example by linking the expression cassette consisting of a promoter operably linked to a first DNA sequence encoding a signal peptide linked in the proper reading frame to a second DNA sequence coding for the desulphatohirudin mutant, and a DNA sequence containing transcription termination signals, or the constituents of the expression cassette to the DNA fragments containing selective genetic markers and origins of replication for the selected microbial host in the predetermined order.

The DNA coding for the desulphatohirudin mutants according to the invention can be manufactured by methods known in the art. The methods for the manufacture of the DNA include excising a portion of the DNA comprising the codons for the undesired amino acid residues from the parental desulphatohirudin gene and replacing it with a DNA segment wherein said codons have been substituted with deoxyribonucleotide triplets coding for the desired amino acid residues, or accomplishing the mutation by means of site-directed mutagenesis.

For the preparation of the DNA coding for the desulphatohirudin mutant, excision of a portion of the desulphatohirudin DNA may be effected by using restriction enzymes. A prerequisite of this method is the availability of appropriate restriction sites in the vicinity of the codons to be altered. For example, a small restriction fragment containing the codon for an undesired amino acid is removed by endonuclease cleavage. A corresponding double stranded DNA sequence is prepared, for example by means of chemical synthesis, in which triplets coding for the desired amino acid are used. The DNA fragment is ligated in the proper orientation to the remaining large fragment to yield a double stranded DNA sequence coding for the mutant. For convenience and in order to facilitate handling of the mutant gene the latter is advantageously contained in a greater DNA segment provided with appropriate linkers which allow insertion and cloning of the segment in a cloning vector.

In a preferred embodiment of the present invention the preparation of DNAs coding for the desulphatohirudin mutants is effected by site-directed mutagenesis. This method is an in vitro mutagenesis procedure by which a defined site within a region of cloned DNA can be altered [cf. the review articles of M.J. Zoller and M. Smith, Methods Enzymol. 100, 468 (1983); D. Botstein and D. Shortle, Science 229, 1193 (1985)]. Mutagenesis can either be effected on the complete desulphatohirudin gene or on functional parts thereof containing the codon(s) for the undesired amino acid(s). After mutagenesis, the mutated functional part is linked to the other parts of the desulphatohirudin gene to yield the complete desulphatohirudin mutant DNA.

The method of mutating the desulphatohirudin gene or functional part thereof is characterized in that the single-stranded gene or a single-stranded DNA comprising the desulphatohirudin gene or part thereof is hybridized to an oligodeoxyribonucleotide primer which is complementary to the region of the hybrid gene to be mutated except for mismatch(es) that direct(s) the mutation, the hybridized oligodeoxyribonucleotide is used as a primer to initiate the synthesis of the complementary DNA strand, the resulting (partially) double-stranded DNA is transformed into a recipient microorganism strain, the microorganism strain is cultivated and transformants containing DNA with the modified (mutant) desulphatohirudin gene are selected.

Transformed microbial hosts

The invention concerns furthermore a microbial host strain which has been transformed with a hybrid vector comprising an expresison cassette consisting of a yeast promoter operably linked to a first DNA sequence encoding a signal peptide linked in the proper reading frame to a second DNA sequence coding for the desulphatohirudin mutant, and a DNA sequence containing transcription termination signals, and to the method for the production thereof, comprising transforming said microbial host strain with said hybrid vector.

The microbial host strains are those specified above. The transformation with the hybrid vectors according to the invention is carried out, for example, in the manner described in the literature for S. cerevisiae [A. Hinnen et al., Proc. Natl. Acad. Sci. USA 75, 1929 (1978)], B. subtilis [Anagnostopoulos et al., J. Bacteriol. 81, 741 (1961)] and E. coli [M. Mandel et al., J. Mol. Biol. 53, 159 (1970)]. The isolation of the transformed host cells is effected advantageously from a selective nutrient medium to which there has been added, for example, the biocide against which the marker gene contained in the expression plasmid imparts resistance. If, for example, the hybrid vectors contain the $amp^R$ gene, ampicillin is accordingly added to the nutrient medium. Cells that do not contain the hybrid vector are destroyed in such a medium.

Pharmaceutical compositions

The novel mutants of desulphatohirudin obtainable in accordance with the present invention have valuable pharmacological properties and can, like hirudin extracted from leeches, by used prophylactically or, especially, therapeutically.

The desulphatohirudin mutants according to the invention are, like natural hirudin, potent inhibitors of thrombin. They have, for example, a $K_i$ (desulphatohirudin mutant-thrombin complex dissociation constant) value of $10^{-9}$ M to $10^{-12}$ M. The desulphatohirudin mutants are completely specific to thrombin and exhibit no interactions with other proteinases of the blood coagulation system. The toxicity is extremely low. Similarly, no hypersensitivity reactions or allergic reactions are observed. Moreover, the mutants of the invention have an in vivo duration of action comparable with or better than that of native desulphatohirudin.

The novel desulphatohirudin mutants according to the invention can therefore be used analogously to natural hirudin for the therapy and prophylaxis of thromboses and thromboembolisms, including the prophylaxis of post-operative thromboses, for acute shock therapy (for example for septic or polytraumatic shock), for the therapy of consumption coagulopathies, in haemodialyses, haemoseparations and in extracorporeal blood circulation.

6

The invention relates also to pharmaceutical compositions that contain at least one of the compounds according to the invention or a pharmaceutically acceptable salt thereof, optionally together with a pharmaceutically acceptable carrier and/or adjuncts.

These compositions can be used especially in the above-mentioned indications, when they are administered, for example, parenterally, such as intravenously, intracutaneously, subcutaneously or intramuscularly, or topically.

The invention relates also to the use of the novel compounds according to the invention and to pharmaceutical compositions containing them for the prophylactic and therapeutic treatment of the human or animal body, especially for the above-mentioned clinical syndromes, especially for inhibiting the coagulation of blood inside and outside the human or animal body.

The dosage depends especially on the specific form of administration and on the purpose of the therapy or prophylaxis. The size of the individual doses and the administration regime can best be determined by way of an individual judgment of the particular case of illness; the methods of determining relevant blood factors required for this purpose are familiar to the person skilled in the art. Normally, in the case of an injection the therapeutically effective amount of the compounds according to the invention is in a dosage range of from approximately 0.005 to approximately 0.1 mg/kg body weight. A range of from approximately 0.01 to approximately 0.05 mg/kg body weight is preferred. The administration is effected by intravenous, intramuscular or subcutaneous injection. Accordingly, pharmaceutical compositions for parenteral administration in single dose form contain per dose, depending on the mode of administration, from approximately 0.4 to approximately 7.5 mg of the compound according to the invention. In addition to the active ingredient these pharmaceutical compositions usually also contain a buffer, for example a phosphate buffer, which is intended to keep the pH value between approximately 3.5 and 7, and also sodium chloride, mannitol or sorbitol for adjusting the isotonicity. They may be in freeze-dried or dissolved form, it being possible for solutions advantageously to contain an antibacterially active preservative, for example from 0.2 to 0.3 % 4-hydroxybenzoic acid methyl ester or ethyl ester.

A composition for topical application can be in the form of an aqueous solution, lotion or gel, an oily solution or suspension or a fat-containing or, especially, emulsified ointment. A composition in the form of an aqueous solution is obtained, for example, by dissolving the active ingredients according to the invention, or a therapeutically acceptable salt thereof, in an aqueous buffer solution of from pH 4 to pH 6.5 and, if desired, adding a further active ingredient, for example an anti-inflammatory agent, and/or a polymeric binder, for example polyvinylpyrrolidone, and/or a preservative. The concentration of the active ingredient is from approximately 0.1 to approximately 1.5 mg, preferably from 0.25 to 1.0 mg, in 10 ml of a solution or 10 g of a gel.

An oily form of administration for topical application is obtained, for example, by suspending the active ingredients according to the invention, or a therapeutically acceptable salt thereof, in an oil, optionally with the addition of swelling agents, such as aluminium stearate, and/or surfactants (tensides) having HLB value ("hydrophilic-lipophilic balance") of below 10, such as fatty acid monoesters of polyhydric alcohols, for example glycerine monostearate, sorbitan monolaurate, sorbitan monostearate or sorbitan monooleate. A fat-containing ointment is obtained, for example, by suspending an active ingredient according to the invention, or a salt thereof, in a spreadable fatty base, optionally with the addition of a tenside having an HLB value of below 10. An emulsified ointment is obtained by triturating an aqueous solution of the active ingredient according to the invention, or a salt thereof, in a soft, spreadable base with the addition of a tenside having an HLB value of below 10. All these forms for topical application can also contain preservatives. The concentration of active ingredient is from approximately 0.1 to approximately 1.5 mg, preferably from 0.25 to 1.0 mg, in approximately 10 g of base.

In addition to the compositions described above that are intended for direct medicinal use in the body of a human or an animal, the present invention relates also to pharmaceutical compositions for medicinal use outside the living body of humans or animals. Such compositions are used especially as anticoagulant additives to blood that is being subjected to circulation or treatment outside the body (for example extracorporeal circulation or dialysis in artificial kidneys), preservation or modification (for example haemoseparation). Such compositions, such as stock solutions or alternatively compositions in single dose form, are similar in composition to the injection compositions described above; however, the amount or concentration of active ingredient is advantageously based on the volume of blood to be treated or, more precisely, on its thrombin content. In this connection it must be borne in mind that the active ingredients according to the invention (in free form) completely deactivate approximately 5 times the amount by weight of thrombin, are physiologically harmless even in relatively large amounts, and are eliminated from the circulating blood rapidly even in high concentrations so that there is no risk of overdose, even, for example, during transfusions. Depending on the specific purpose, the suitable dose is from approximately 0.01 to approximately 1.0 mg of the active ingredient/litre of blood, although the upper limit may still be exceeded without risk.

The invention relates especially to the desulphatohirudin mutants, hybrid vectors, microbial host strains transformed with such hybrid vectors, and to the processes for their manufacture as described in the Examples.

Brief description of the drawings

In the following experimental section various embodiments of the present invention are described with reference to the accompanying drawings in which:

Fig. 1 schematically illustrates the construction of plasmid pML350.

Fig. 2 schematically shows the plasmid map of plasmid pJDB207/GAPFL-HIR.

Fig. 3 schematically illustrates the construction of plasmid pJDB207/GAPFL-HIR (Asp[63])

Experimental section

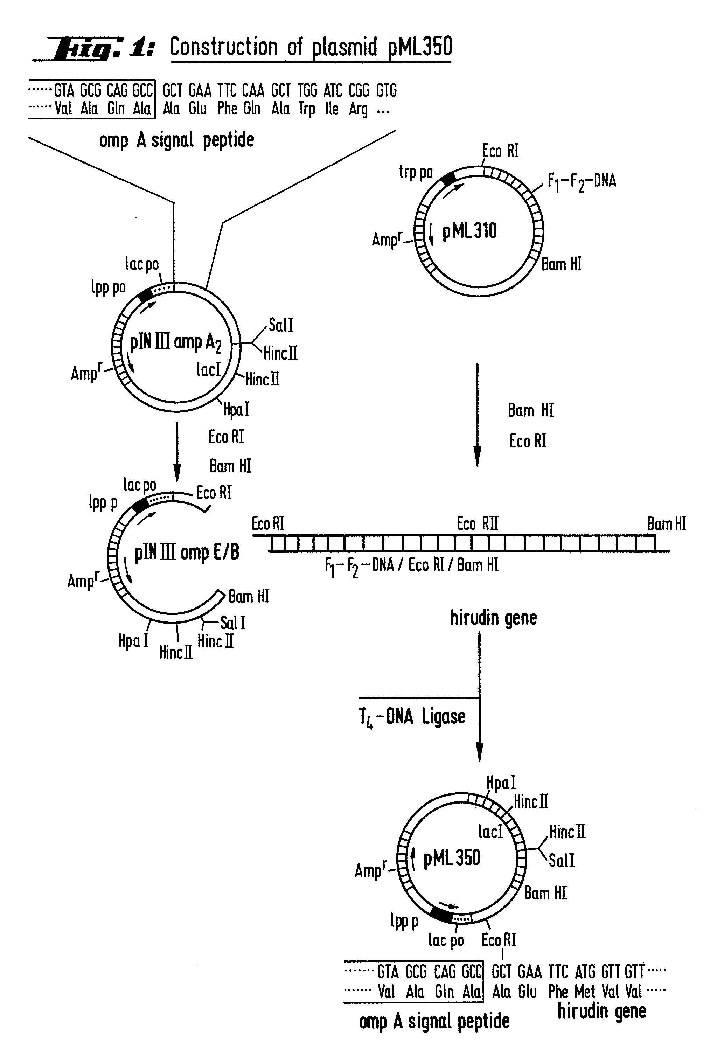

Example 1: Construction of the plasmid pML350 (see Fig. 1)

a) Digestion of the DNA of plasmid pIN-III-ompA-2

10 μg of plasmid pIN-III-ompA-2 [J. Ghrayeb et al., EMBO-J. 3, 2437 (1984)] are dissolved in 25 μl of 100 mM Tris-HCl pH 7.5, 50 mM NaCl and 100 μg/ml gelatine and are digested with the restriction endonucleases EcoRI and BamHI. The solution is adjusted to TNE and extracted with phenol/chloroform. The DNA is precipitated with ethanol. The vector DNA pIN-III-ompA-2/EcoRI/BamHI is isolated after electrophoresis in agarose by gel elution.

b) Digestion of the DNA of plasmid pML310

20 μg of the plasmid pML310 (see European Patent Application No. 168342) are digested in 50 μl of 100 mM Tris-HCl pH 7.5, 50 mM NaCl and 100 μg/ml gelatine with the restriction endonucleases EcoRI and BamHI. The solution is adjusted to TNE and extracted with phenol/chloroform. The DNA is precipitated with ethanol. The $F_1$-$F_2$-DNA (hirudin gene) is isolated after gel electrophoresis in agarose by gel elution.

c) Ligation of the $F_1$-$F_2$-DNA (hirudin gene) from pML310 with the vector DNA pIN-III-ompA-2/EcoRI/BamHI

1 μg of $F_1$-$F_2$-DNA (hirudin gene)/EcoRI/BamHI and 30 μg of the vector DNA pIN-III-ompA-2/EcoRI/BamHI are dissolved in 50 μl of 100 mM Tris-HCl pH 7.5, 50 mM NaCl and 100 μg/ml gelatine, adjusted to TNE. The solution is extracted with phenol/chloroform and the DNA is precipitated with ethanol. The DNA precipitate is dissolved in 20 μl of a solution of 50 mM Tris-HCl (pH 7.8), 10 mM $MgCl_2$, 10 mM DTT, 0.5 mM ATP, and 100 μg/l gelatine and treated with 25 units/μl $T_4$ DNA ligase (Biolabs) at 15°C for 3 h. By this way the recombinant plasmid pML350 is created, which contains the $F_1$-$F_2$-DNA (hirudin gene) inserted.

d) Transformation of E. coli HB101 with plasmid pML350

The E. coli HB101 cells pretreated with calcium that are prepared as described by Mandel et al. [J. Mol. Biol. 53, 159 (1970)].

The solution obtained in c), which contains the recombinant plasmid pML350, is heated at 65°C for 10 min in order to inactivate the $T_4$ DNA ligase and is then cooled to 37°C. 10 μl of the resulting reaction mixture are added to 150 μl of calcium-treated E. coli HB101 cells in 10 mM $MgCl_2$ and 10 mM Tris·HCl (pH 7.5) in a total volume of 200 μl.

Subsequently, this mixture is cooled in ice for 30 min, heated for 2 min at 42°C and then left to stand for 50 min in 1 ml of L-medium (Bacto tryptone 10 g/l; Bacto yeast extract 5 g/l; NaCl 5 g/l; glucose 5 g/l; ampicillin 0.1 g/l) at 37°C. The mixture is then spread out in aliquots of 0.2 ml on 5 agar plates (McConkey Agar, Difco), which contain 60 μg/ml of ampicillin (Serva). The agar plates are then maintained at 37°C for 16-18 hours. 185 ampicillin resistant colonies of transformed E. coli HB101 cells are obtained.

e) Screening the colonies that contain $F_1$-$F_2$-DNA

6 transformed colonies (Example 1d) are pressed off onto nitrocellulose filter B85 (Schleicher and Schull). In accordance with Grunstein and Hogness [Proc. Natl. Acad. Sci. USA 72, 3961 (1979)] the colonies are lysed and their denatured DNA is fixed on the filter. Subsequently prehybridization of the filters is carried out in 20 ml (per filter) of 4xSET [= solution of 30 mM Tris·HCl (pH 8), 150 mM NaCl, 1 mM EDTA], 0.1 % (w/v) Ficoll 400 (Pharmacia), 0.5 % SDS, 50 μg/ml denatured calf thymus DNA for 4 h at 64°C. Subsequently the nitrocellulose filters are treated in 20 ml (per filter) of 5xSET (w/v), 0.1 % (w/v) Ficoll 400, 0.2 % SDS and 50 μl/ml denatured calf thymus DNA for 16 h at 64°C with the $^{32}$P radioactively labelled probe (approximately $10^3$-$10^4$ Cerencov cpm per filter). A mixture consisting of oligonucleotides 46/64 complementary, 1/58, 96/67 and 154/64 complementary (see European Patent Application No. 168342) is used as probe.

Subsequently, the filters are washed twice in 2xSET, 0.2 % SDS at room temperature, then twice in 2xSET, 0.5 % SDS at 60°C (first for 30 min, then for 60 min). The filters are then dried between 3 MM paper (Whatman) and placed at -80°C on an X-ray film (Fuji) with an intensifying screen (Ilford) for 1-2 days.

The resulting autoradiogram shows 5 positive colonies (clones) which can be used for further processing, one of which received the designation pML350.

Example 2: Construction of plasmid pBH109

As plasmid pML350 originates from plasmid pIN-III-ompA-2 including a multi-cloning linker (EcoRI, HindIII, BamHI sites) it contains 12 additional base pairs in front of the mature hirudin gene coding for Ala, Gln, Phe, Met. To get mature desulphatohirudin expressed these 12 base pairs are looped out by in vitro mutagenesis making use of a 27mer oligonucleotide.

a) Preparation of pML350/Xbal/BamHI (SI)

5 μg of plasmid pML350 are digested with the endonucleases Xbal and BamHI. The larger of the two Xbal-BamHI fragments (SI) is isolated by gel elution after electrophoresis on agarose and dissolved in 1 mM Tris-HCl pH 7.5, 0.1 mM EDTA.

b) Preparation of pML350/Pvul (SII)

5 μg of plasmid pML350 are digested with the endonuclease Pvul. The linearized DNA pML350/Pvul is subsequently digested with 3 units of intestinal alcaline phosphatase (Boehringer) for 30 min at 37°C. The enzyme is inactivated by heating the solution for 60 min at 65°C. The linear pML350/Pvul (SII) DNA is isolated by gel elution after electrophoresis on agarose and dissolved in 1 mM Tris-HCl pH 7.5, 0.1 mM EDTA.

c) Preparation of the oligonucleotide (27 mer) I27

In analogy to the procedure described in European Patent Application No. 168342 the following DNA fragment (designated I27) has been synthesized:

5'-GTA GCG CAG GCC GTT GTT TAC ACC GAC-3'

<u>I27</u>

The phosphorylation at the 5' ends was done with [γ-$^{32}$P]-ATP and T$_4$ polynucleotide kinase (Boehringer) as described [Molecular Cloning, A Laboratory Manual (ed. T. Maniatis et al.), Cold Spring Harbor Lab. (1982), p. 125].

d) Construction of plasmid pBH109

0.3 μg each of SI DNA and of SII DNA are mixed with 40 pmol of the phosphorylated DNA fragment I27 in 27 μl of 1 mM Tris-HCl pH 7.5, 0.1 mM EDTA. To the mixture 3 μl of 10X polymerase-ligase buffer (1M NaCl, 65 mM Tris-HCl pH 7.5, 80 mM MgCl$_2$ and 10 mM β-mercaptoethanol) are added. This mixture is heated for 3 min in a boiling water bath to denature the DNA fragments. Subsequently, the mixture is gradually cooled (about 1°C/min) to 30°C and incubated at this temperature for 30 min. Further the mixture is incubated at 4°C for 30 min and afterwards for 10 min in ice.

12 μl of the four deoxyribonucleotide phosphates (7.5 mM each), 6 μl of 10 mM ATP, 6 μl of T$_4$ DNA ligase (2.5 U/μl) and 1.2 μl of Klenow DNA polymerase (Boehringer, 5 U/μl) are added and the DNA mixture (total volume 55 μl) is incubated for 16 h at 12.5°C.

The DNA mixture is digested with 2 units of endonuclease EcoRI for 1 h at 37°C in order to destroy unchanged starting plasmid pML350. With this procedure, plasmid pBH109 is formed. Plasmid pBH109 contains the lpp promoter and the lac promoter/operator operably linked to the ompA-2 signal sequence linked in frame to the gene coding for mature desulphatohirudin.

e) Transformation of E. coli HB101 with plasmid pBH109

The transformation with calcium treated <u>E. coli</u> HB101 cells is done as described in Example 1d. The total reaction mixture used is 55 μl.

f) Screening of the colonies which contain plasmid pBH109

100 transformed colonies are cultivated, from each colony plasmid DNA is prepared and digested with EcoRI. All plasmid DNAs, which are not digestable with EcoRI are potent plasmids pBH109 which is lacking the EcoRI site.

Two positive identical colonies have been identified. One of them is selected and designated pBH109.

The correct sequence of the F$_1$-F$_2$-DNA following the ompA-2 leader sequence is confirmed by sequence analysis.

Example 3: Mutation of the residues Glu61, Glu62 of hirudin to Gln61, Gln62 using single stranded M13mp19/hirudin.

EP 0 352 227 A2

```
                                56        58        60  61  62        64
coding strand
                              Phe Glu Glu Ile Pro Glu Glu Tyr Leu Glu
of hirudin
                         5'...TTC GAA GAA ATC CCG GAA GAA TAC CTG CAG 3'


mutagenic primer 1        3'        CTT CTT TAC GGC GTT GTT ATG GAC GTC 5'

mutated coding

strand                   5'...TTC GAA GAA ATC CCG CAA CAA TAC CTG CAG 3'

                              Phe Glu Glu Ile Pro Gln Gln Tyr Leu Gln
```

Mutagenic primers are synthesised using the phosphoramidite method [M.H. Caruthers, in Chemical and Enzymatic Synthesis of Gene Fragments (H.G. Gassen and A. Lang, Eds.) Verlag Chemie, Weinheim, Federal Republic of Germany] on an Applied Biosystems (Model 380B) synthesiser.

I. Preparation of M13mp19/hirudin

A. XbaI-BamHI cut M13mp19 DNA.
To 5 µl M13mp19 double stranded DNA (ds-DNA; 0.1 µg/ml; BRL) are added 2 µl React 2 (500 mM Tris-HCl, pH 8.0; 100 mM MgCl₂, 500 mM NaCl) (BRL), 1 µl XbaI (10 U/µl), 0.5 µl BamHI (10 U/µl) and 12 µl H₂O. After incubation at 37°C for 1.5 h, 0.5 µl BamHI, 2.5 µl React 3 (500 mM Tris-HCl, pH 8.0; 100 mM MgCl₂; 1000 mM NaCl) (BRL), and 2 µl H₂O are added and incubation is continued at 37°C for 1 h. The volume is made up to 100 µl with H₂O. The ds-DNA is isolated by phenol extraction and ethanol precipitation, and dissolved in 30 µl of TE buffer (Tris-HCl 10 mM, EDTA 1 mM, pH 8.0).

B. Insert DNA.
Five 5 µg of the plasmid pBH109 are cut with XbaI and BamHI as described above and the digest is electrophoresed for 3 h at 150 volt using a 3.5 % polyacrylamide gel with 1x TBE buffer (10x TBE buffer: 108 g Tris, 55 g boric acid, 9.3 g EDTA·2H₂O/l). The XbaI-BamHI fragment containing the hirudin gene (250 bp) is visualised under UV light after immersing the gel in 400 ml 1x TBE buffer containing 10 µl of ethidium bromide solution (10 µg/ml in water). The part of the gel containing the restriction fragment is cut from the gel and placed in a dialysis bag with 500 µl of 0.5x TBE, and the DNA is electroeluted in a BIO-RAD minigel electrophoresis apparatus using 0.5x TBE as the running buffer at 170 volt for 30 min. The DNA is loaded onto an Elutip-d column (Schleicher & Schull) equilibrated with 0.5x TBE. The column is washed with 2 ml of 0.5x TBE and the DNA is eluted with 1 M NaCl in 0.5x TBE (1 ml). The DNA is precipitated with ethanol and redissolved in 10 µl of TE buffer.

C. Ligation of XbaI-BamHI hirudin insert into M13mp19 and preparation of single stranded DNA.
Five µl XbaI-BamHI hirudin insert, 2 µl XbaI-BamHI cut M13mp19, 1 µl 10x ligase buffer (50 mM Tris-HCl, pH 7.5, 10 mM MgCl₂, 10 mM dithiothreitol), 1 µl ATP, 1.5 µl T4 DNA ligase (BRL; 1 U/µl) are mixed and incubated overnight at 14°C. Five µl of ligation mixture are used to transform E. coli JM101 competent cells according to the method of J. Messing [Methods in Enzymology 101, 21-78 (1983)]. Twelve clear plaques are picked and single stranded DNA (ss-DNA) is prepared from each plaque as described by J. Messing (supra). The DNA designated M13mp19/hirudin is redissolved in 50 µl of TE buffer (0.1-0.5 µg/µl).

II. Site-directed mutagenesis.

A. Phosphorylation of mutagenic primer.
200 pmol (23 µl) of mutagenic primer 1 (see above) is phosphorylated by adding 3 µl 10x kinase buffer (1M Tris-HCl, 0.1M MgCl₂, 0.1M dithiothreitol, pH 8.3) 3 µl 10 mM ATP and 1 µl T4 polynucleotide kinase (BRL, 10 U/µl). After incubation at 37°C for 1 h, the reaction is stopped by heating at 65°C for 10 min.

B. Annealing of the mutagenic primer 1 to the single-stranded M13mp19/hirudin template.
Six µl (0.5 µg) of single-stranded M13mp19/hirudin (Section I-C) is incubated with 3 µl (20 pmol) of the phosphorylated mutagenic oligodeoxyribonucleotide (6.6 pmol/µl) and 1 µl buffer A (0.2M Tris-HCl, pH 7.5, 0.1M MgCl₂, 0.5M NaCl, 0.01M DTT) at 70°C for 5 min, and cooled slowly to room temperature over 30 min.

C. Extension-ligation reaction.
To the above annealed mix is added 1 µl buffer B (0.2M Tris-HCl, pH 7.5, 0.1M MgCl₂, 0.01M DTT), 1 µl 10mM ATP, 4 µl 2mM dNTP mixture, 5 µl T4 DNA polymerase (Boehringer, 1 U/µl), 5 µl T4 DNA ligase (BRL, 1 U/µl). This mixture is incubated at 16°C for 3 h. The reaction is stopped by incubating at 65°C for 10 min.

## D. Transformation and preparation of single-stranded mutant DNA

The ligation mixture is diluted 1:20 with sterile $H_2O$, and 1 µl, 5 µl, as well as 1 µl undiluted ligation mixture is used to transform competent E. coli BMH 71-81 mut S cells [B. Kramer, W. Kramer and H.-J. Fritz, Cell 38, 879-887 (1984)]. The cells are plated out as described in the "M13 cloning and sequencing Handbook" (published by Amersham). Twelve colourless plaques are picked and ss-DNA is prepared as described in section I-C.

## E. Screening of single-stranded DNA for mutant.

To screen for mutated single-stranded DNA, each of the 12 ss-DNA samples is sequenced by the dideoxynucleotide chain termination method [F. Sanger, S. Nickler and A.R. Coulson, Proc. Natl. Acad. Sci. USA 74, 5463-5467 (1977)]. Initially, only the dideoxynucleotide complementary to the expected mutated base is used in the reaction. Subsequently, the ss-DNA from several positive mutants are sequenced to establish the full DNA sequence of the mutant using T7 DNA polymerase (Sequenase, USB) following the method of Tabor and Richardson [Proc. Natl. Acad. Sci. USA 84, 4767-4771 (1987)]. The expected base changes encoding Glu -> Gln mutation at positions 61 and 62 of the recombinant hirudin are observed in the DNA sequence. The phage DNA having the expected sequence is designated M13mp19/hirudin E61,62Q.

## F. Preparation of replicative form (RF) DNA from single-stranded M13mp19/hirudin E61,62Q phage DNA.

Competent E.coli JM101 cells are transformed with 10-20 ng of single-stranded hirudin E61,62Q mutant DNA and ds-DNA is prepared as described in the "M13 cloning and sequencing Handbook" (published by Amersham). A yield of 40-50 µg of ds-DNA is obtained from 100 ml of culture.

## G. Isolation of mutant hirudin XbaI-BamHI insert.

The mutated hirudin XbaI-BamHI insert is cut out of 25 µg of the ds-DNA and purified as described in section IB. The DNA is dissolved in 20 µl of sterile water.

## H. Preparation of XbaI-BamHI cut pIN-III-ompA-2 vector DNA

A digest of approximately 1.5 µg pIN-III-ompA-2 plasmid is made by adding 6 µl React 2 buffer (BRL), 2 µl (20 Units) XbaI, 1 µl BamHI (10 Units), 1 µl EcoRI (10 Units) and 37 µl $H_2O$ (total volume 50 µl), and incubation for 3 h at 37°C. 1 µl (10 units) BamHI, 1 µl (10 Units) EcoRI, 5 µl React 3 (BRL) and 12 µl $H_2O$ are added and incubation continued for 1 h at 37°C.

## I. Ligation of mutant hirudin E61,62Q XbaI-BamHI insert DNA into XbaI-BamHI cut pIN-III-ompA-2 plasmid.

Nine µl hirudin E61,62Q XbaI-BamHI insert DNA, 2 µl XbaI-BamHI cut pIN-III-ompA-2 vector DNA, 3 µl 10x ligation buffer (BRL) and 1 µl (1 U/µl) T4 DNA ligase (BRL) are mixed and incubated at 14°C for 16-20 h.

## III. Expression of mutant [Gln61,62]-desulphatohirudin in E. coli JM101.

### A. Transfection into E. coli strain JM101.

Five µl of ligation mixture is used to transform 0.3 ml of E. coli JM101 competent cells according to the method of J. Messing (supra). Three ml of 2xYT/Ampicillin (50 µg ampicillin/ml 2xYT) is added to the sample and the cells allowed to grow at room temperature for 1 h. A 1 ml sample of the culture is then taken and poured onto an LB/Ampicillin (50 µg ampicillin/ml LB-agar) plate and grown overnight at 37°C.

### B. Selection of [Gln61,62]-desulphatohirudin expressing colonies.

Ten bacterial colonies from the LB/Ampicillin plates are picked and grown separately for 5 h at 37°C in 5ml LB/Ampicillin (50 µg Ampicillin/ml LB). 1 ml samples are taken from each culture tube and the cells recovered by centrifugation (3000xg for 5 min). Each sample of cells is osmotically shocked by treatment with 100 µl of 10 mM Tris-HCl, pH 8.1 for 30 min at 0°C to release the material in the periplasmic space of the bacteria. The cells are removed by centrifugation as before, and the supernatant is tested for hirudin activity as described in Section V-B. The sample which gives the highest inhibitory activity is selected for batch culture.

### C. Batch culture and isolation of [Gln61,62]-desulphatohirudin.

The remaining quantity (4 ml) of cells from the most active sample is used to inoculate 1 l of LB/Ampicillin (50 µg Ampicillin/ml LB). The culture is grown overnight at 37°C, and the cells are recovered by centrifugation (3000xg for 15 min.). The cell pellet is osmotically shocked by resuspension in 50 ml of 10 mM Tris-HCl, pH 8.1 at 0°C for 1 h. The cells are removed from the periplasmic fraction by centrifugation at 6000xg for 10 min.

### D. Purification of [Gln61,62]-desulphatohirudin.

The pH of the periplasmic fraction is adjusted to 6.5 with 0.1M HCl and filtered through a 0.45 µm filter (Nalgene). The protein is loaded onto a Mono-Q column FPLC system (Fast Protein Liquid Chromatography, Pharmacia-LKB) equilibrated with 50mM bis-Tris-HCl buffer pH 6.5. The desulphatohirudin mutant is eluted from the column using a linear salt gradient of 0-300 mM NaCl in bis-Tris-HCl pH 6.5 over 45 min. 0.8 ml fractions of the column eluate are collected and tested for hirudin activity as described in Section III-B. The desulphatohirudin mutant-containing fractions are pooled, filtered as above and chromatographed on a Millipore-Waters HPLC system using a Brownlee Labs C8 reversed-phase HPLC column equilibrated with 0.09 % (v/v) trifluoroacetic acid in $H_2O$. The hirudin mutant is eluted from the column with a linear gradient of 7 to 28 % (v/v) acetonitrile in 0.09 % (v/v) trifluoroacetic acid in $H_2O$. [Gln$^{61,62}$]-desulphatohirudin having a purity of about or more than 98 % elutes as a single peak at 28 min.

E. Protein chemical characterization

Purified [Gln$^{61,62}$]-desulphatohirudin has been characterized by determining its amino acid composition, N-terminal sequence, and by peptide mapping.

Two to five μg protein are hydrolysed in glass tubes in the vapor of 6N HCl at 110°C [R.L. Henrikson, and S.C. Meredith, Anal. Biochem. 136, 65-74 (1984)]. The amino acids are dried under vacuum, derivatized with phenylisothiocyanate, and separated on a reversed phase column [B.A. Bidlingmeyer, S.A. Cohen and T.L. Taruin, J. Chromatography 336, 93-104 (1984)]. The amino acid composition is calculated using a molecular weight of 6889 D.

Approximately 50 pmol of [Gln$^{61,61}$]-desulphatohirudin are subjected to 5 cycles of automated Edman degradation, using an Applied Biosystems 470A gas phase sequencer [M.W. Hunkapiller and L.E. Hood, Methods in Enzymology 91, 486-494, (1983)], to establish the N-terminal amino acid sequence. The resulting phenylthiohydantoin amino acids are separated on a reversed phase HPLC system [R.M. Hewick, M.W. Hunkapiller, L.E. Hood and W.J. Dreyer, J. Biol. Chem. 256, 7990-7997 (1981)], using an Applied Biosystems type 120 on-line analyser.

To obtain a peptide map of [Gln$^{61,62}$]-desulphatohirudin, 50 μg of protein are dried under vacuum, and treated with performic acid for 1 h at 37°C [C.H.W. Hirs, Methods in Enzymology 11, 197-199 (1967)]. 45 μl of cold water are added to the reaction mixture, frozen and dried under vacuum (twice). The oxidized hirudin mutant is dissolved in 50 μl of 50 mM $NH_4HCO_3$ and digested with 1 μg thermolysin (Boehringer) for 4 h at 37°C. Prior to fractionation, the digest is dried under vacuum, and dissolved in 0.1 % trifluoroacetic acid (v/v) in $H_2O$. Peptides are purified by reversed phase HPLC, using a Vydac C18 column, equilibrated in 0.1 % trifluoroacetic acid in $H_2O$. Peptides are eluted with a linear gradient of 0-28 % acetonitrile over 90 min, at a flow rate of 1 ml/min.

The amino acid sequence of the peptide containing the mutations is sequenced as described above to confirm the amino acid substitutions Glu61, Glu62 → Gln61, Gln62.

Example 4: Mutation of the residues Gln 61,62 of [Gln$^{61,62}$]-desulphatohirudin to Leu 61,62

```
                        56        58        60        62        64
coding strand           Phe  Glu  Glu  Ile  Pro  Gln  Gln  Tyr  Leu  Gln

of hirudin           5'TTC  GAA  GAA  ATC  CCG  CAA  CAA  TAC  CTG  CAG 3'


mutagenic

primer 2             3'     CTT  CTT  TAG  GGC  GAT  GAT  ATG  GAC  GTC 5'

mutated              5'TTC  GAA  GAA  ATC  CCG  CTA  CTA  TAC  CTG  CAG 3'

coding strand           Phe  Glu  Glu  Ile  Pro  Leu  Leu  Tyr  Leu  Gln
```

Site-directed mutagenesis is performed using mutagenic primer 2 and M13mp19/hirudin E61,62Q exactly as described in Example 3 (Sections I and II). The mutant protein is expressed in E. coli and purified as described for Example 3 (Section III). The identity of the mutant protein in which Gln 61,62 are replaced by Leu 61,62 is confirmed by amino acid composition and N-terminal sequence analysis (Example 3; Section E) and its inhibitory properties are determined (Example 14). The mutant is designated [Leu$^{61,62}$]-desulphatohirudin.

Example 5: Mutation of the residues Glu 61,62 of hirudin to Asn 61,62

```
                        56       58       60       62       64
coding strand          Phe Glu Glu Ile Pro Glu Glu Tyr Leu Gln
of hirudin          5'TTC GAA GAA ATC CCG GAA GAA TAC CTG CAG 3'


mutagenic
primer 3            3'   CTT CTT TAG GGC TTA TTA ATG GAC GTC 5'


mutated             5'TTC GAA GAA ATC CCG AAT AAT TAC CTG CAG 3'
coding strand          Phe Glu Glu Ile Pro Asn Asn Tyr Leu Gln
```

The procedures given above for Examples 3 and 4 are repeated using mutagenic primer 3 to obtain and characterize the desired mutant protein in which Glu 61,62 are replaced by Asn 61,62. The mutant is designated [Asn[61,62]]-desulphatohirudin.

### Example 6: Mutation of the residues Glu 57,58,61,62 of hirudin to Leu 57,58,61,62

```
                            56       58       60       62       64
coding strand    Gly Asp Phe Glu Glu Ile Pro Glu Glu Tyr Leu Gln
of hirudin    5'GTT GAC TTC GAA GAA ATC CCG GAA GAA TAC CTG CAG 3'


mutagenic
primer 4      3'CCA CTG AAG GAT GAT TAG GGC GAT GAT ATG GAC GTC 5'


mutated       5'GGT GAC TTC CTA CTA ATC CCG CTA CTA TAC CTG CAG 3'
coding strand    Gly Gly Phe Leu Leu Ile Pro Leu Leu Tyr Leu Gln
```

The procedures given above for Examples 3 and 4 are repeated using mutagenic primer 4 to obtain and characterize the desired mutant protein in which Glu 57,58,61,62 are replaced by Leu 57,58,61,62. The mutant is designated [Leu[57,58,61,62]]-desulphatohirudin.

### Example 7: Mutation of the residues Glu 57,58,61,62 of hirudin to Asn 57,58,61,62

```
                 54       56       58       60       62       64
coding strand    Gly Asp Phe Glu Glu Ile Pro Glu Glu Tyr Leu Gln
of hirudin    5'GGT GAC TTC GAA GAA ATC CCG GAA GAA TAC CTG CAG 3'


mutagenic
primer 5      3'CCA CTG AAG TTA TTA TAG GGC TTA TTA ATG GAC GTC 5'


mutated       5'GGT GAC TTC AAT AAT ATC CCG AAT AAT TAC CTG CAG 3'
coding strand    Gly Asp Phe Asn Asn Ile Pro Asn Asn Tyr Leu Gln
```

The procedures given above for Examples 3 and 4 are repeated using mutagenic primer 5 to obtain and characterize the desired mutant protein in which Glu 57,58,61,62 are replaced by Asn 57,58,61,62. The mutant is designated [Asn$^{57,58,61,62}$]-desulphatohirudin.

Example 8: Mutation of residue Asp 53 of hirudin to Ala 53

```
                        50        52        54        56
coding strand     Ser His Asn Asp Gly Asp Phe Glu
of hirudin        5'TCT CAC AAC GAC GGT GAC TTC GAA 3'


mutagenic
primer 6          3'AGA GTG TTG CGC CCA CTG AAG CTT 5'


mutated           5'TCT CAC AAC GCC GGT GAC TTC GAA 3'
coding strand     Ser His Asn Ala Gly Asp Phe Glu
```

The procedures given above for Examples 3 and 4 are repated using mutagenic primer 6 to obtain and characterize the desired mutant protein in which Asp 53 is replaced by Ala 53. The mutant is designated [Ala$^{53}$]-desulphatohirudin.

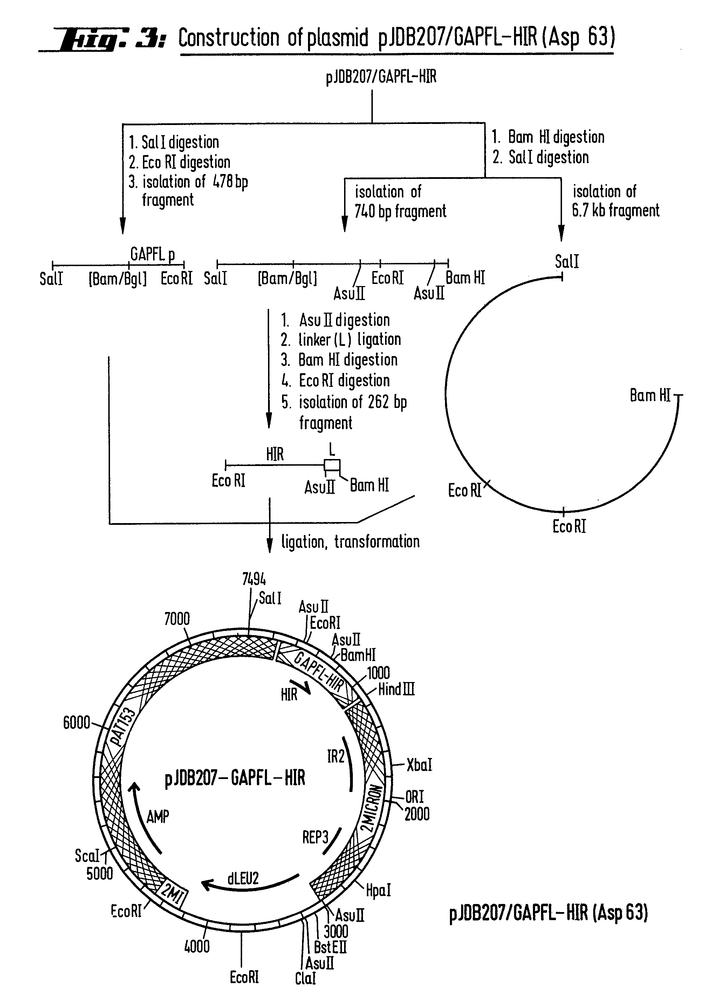

Example 9: Mutation of the residue Tyr 63 of desulphatohirudin to Asp 63 or Glu 63 (see Fig 3)

The mutation of the aminoacid residue Tyr 63 of desulphatohirudin to Asp or Glu is achieved by insertion into the coding sequence of an oligonucleotide linker which has the TAC codon for Tyr 63 replaced by GAC (coding for Asp) or GAA coding (for Glu). The resulting polypeptides are referred to as [Asp$^{63}$]-desulphatohirudin and [Glu$^{63}$]-desulphatohirudin, respectively.

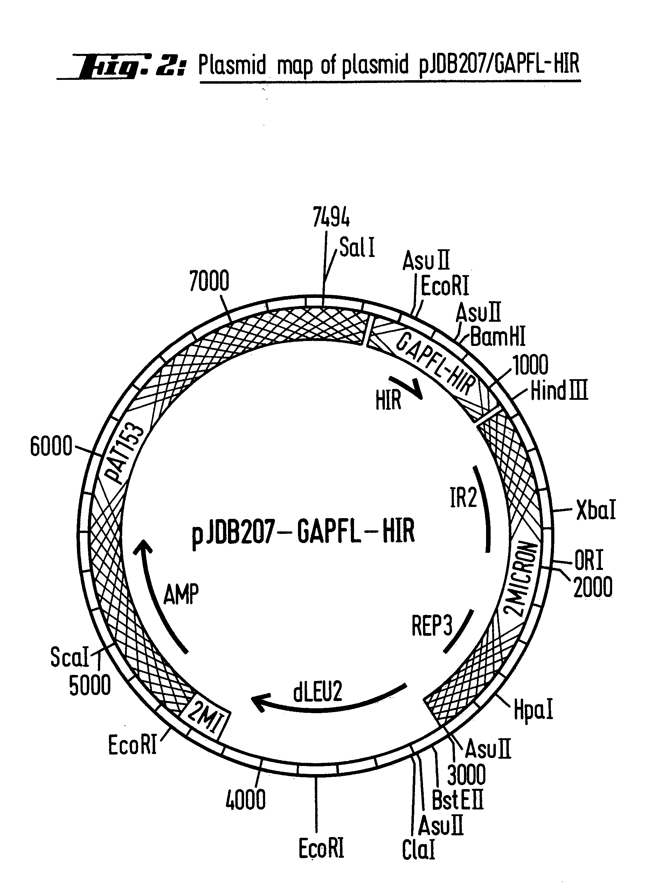

Yeast expression plasmid pJDB207/GAPFL-HIR (see Fig. 2; European patent application No. 225633) is digested with restriction endonucleases Sall and EcoRI. The 478 bp Sall-EcoRI fragment is separated on a 0.8 % preparative agarose gel in TBE buffer (90 mM Tris-base, 90 mM boric acid, 2.5 mM EDTA pH 8.3). The ethidiumbromide-stained fragment is isolated from the gel. The DNA is electroeluted in 0.2xTBE buffer for 45 min at 100 mA and purified by DE52 (Whatman) ion exchange chromatography. The DNA is eluted from the DE52 column with a high salt buffer (1.5 M NaCl, 10 mM Tris-HCl pH 8.0, 1 mM EDTA), precipitated with ethanol and redissolved in H$_2$O at a concentration of 0.1 pmoles/μl. The 478 bp Sall-EcoRI fragment contains the Sall-BamHI sequence of pBR322 fused to the BglII-EcoRI GAPFL promoter fragment.

Plasmid pJDB207/GAPFL-HIR is digested with BamHI and Sall. The large, 6.7 kb vector fragment is isolated as described above. The small, 740 bp Sall-BamHI fragment is also isolated. It contains the sequence of the 478 bp Sall-EcoRI fragment (see above) in addition to the PH05 signal sequence fused in frame to the coding sequence of desulphatohirudin. The 740 bp fragment is digested with AsuII. The DNA is extracted with phenol/chloroform, precipitated with ethanol and redissolved in H$_2$O.

Synthetic oligodeoxynucleotides of the formula

```
                         Asp
(1) 5'-CGAAGAAATCCCGGAAGAAGACCTGCAGTAG      -3'
(2) 3'-  TTCTTTAGGGCCTTCTTCTGGACGTCATCCTAG -5'


                         Glu
(3) 5'-CGAAGAAATCCCGGAAGAAGAACTGCAGTAG      -3'
(4) 3'-  TTCTTTAGGGCCTTCTTCTTGACGTCATCCTAG -5'
```

are each kinased in 40 μl of 60 mM Tris-HCl pH 7.5, 10 mM MgCl$_2$, 5 mM DTT, 0.5 mM ATP and 27 U of T4 polynucleotide kinase (Boehringer) for 45 min at 37°C. The reaction mixtures for oligonucleotides (1) and (2) are combined as well as the reaction mixtures for (3) and (4). Both mixtures are heated for 10 min at 75°C and allowed to cool to room temperature. The annealed oligonucleotide linkers (1+2) and (3+4) are stored at -20°C.

14

0.85 µg (3.8 pmoles) of the AsuII digested DNA are incubated for 16 h at 15°C with a 100 fold excess of the kinased and annealed oligonucleotide linker over DNA ends in 150 µl of 60 mM Tris-HCl pH 7.5, 10 mM MgCl₂, 5 mM DTT, 3.5 mM ATP and 1200 U of T4 DNA ligase (Biolabs). After inactivation of the T4 DNA ligase for 10 min at 85°C the excess linkers are removed by precipitation of the DNA in the presence of 10 mM EDTA, 300 mM sodium acetate pH 6.0 and 0.54 volumes of isopropanol. The DNA is digested with EcoRI and BamHI. The resulting fragments are separated on a 2 % preparative agarose gel in TBE buffer. The 262 bp fragment is recovered from the gel by electroelution and ethanol precipitation. The DNA is resuspended at a concentration of 0.1 pmoles/µl. The EcoRI-BamHI fragment contains the coding sequence of desulphatohirudin with the GAC or GAA triplets coding for Asp 63 or Glu 63, respectively.

Three DNA fragments, isolated as described above, are ligated in the following reaction: 0.2 pmoles of the 478 bp SalI-EcoRI fragment, 0.2 pmoles of the 262 bp EcoRI-BamHI fragment (containing sequence GAC or GAA, respectively) and 0.1 pmoles of the 6.7 kb vector fragment are ligated in 10 µl of 60 mM Tris-HCl pH 7.5, 10 mM MgCl₂, 5 mM DTT, 1 mM ATP and 200 U of T4 DNA ligase for 6 h at 15°C. A one µl aliquot of each ligation mixture is added to 100 µl of calcium-treated, transformation competent E. coli HB101 cells.

12 Transformed, ampicillin-resistant colonies are grown in LB medium containing 100 mg/l of ampicillin. Plasmid DNA is prepared [Holmes et al., Anal. Biochem. 114 (1981), 193] and analysed by BamHI, EcoRI double digests. The presence of the mutations in the DNA is confirmed by DNA sequencing [Sanger et al., Proc. Natl. Acad. Sci. USA 74 (1977) 5463]. One clone with the GAC codon in the hirudin structural gene is referred to as pJDB207/GAPFL-HIR (Asp 63). One clone with the GAA sequence coding for Glu at position 63 of hirudin is referred to as pJDB207/GAPFL-HIR (Glu 63).

## Example 10: Construction of a yeast expression plasmid coding for [Pro66]-desulphatohirudin

The DNA sequence coding for desulphatohirudin is extended by the oligonucleotide CCA, which codes for proline. The resulting new desulphatohirudin is expressed in yeast. It contains 66 aminoacids with a proline at its C-terminal end. This polypeptide is referred to as [Pro66]-desulphatohirudin.

Plasmid pJDB207/GAPFL-HIR (Pro 66) is constructed in the same way as described in Example 9 except for using the following oligodeoxynucleotides:

<div align="center">Pro</div>

```
(5) 5'-CGAAGAAATCCCGGAAGAATACCTGCAGCCATAG      -3'

(6) 3'-  TTCTTTAGGGCCTTCTTATGGACGTCGGTATCCTAG -5'
```

A single DNA clone, which has the CCA extension in the hirudin coding sequence is referred to as pJDB207/GAPFL-HIR (Pro 66).

## Example 11: Transformation of Saccharomyces cerevisiae strains GRF18 and HT246

Saccharomyces cerevisiae strains GFR18 (DSM 3665; α, his3-11, his3-15, leu2-3, leu2-112, can^R) and HT246 (a, leu2-3, leu2-112, prb) (DSM 4084) are transformed with the plasmids
pJDB207/GAPFL-HIR (Asp 63)
pJDB207/GAPFL-HIR (Glu 63)
pJDB207/GAPFL-HIR (Pro 66)
using the transformation protocol described by Hinnen et al. [Proc. Natl. Acad. Sci. USA 75, 1929 (1978)]. Transformed yeast cells are selected on yeast minimal media plates deficient in leucine. Single transformed yeast colonies are isolated and referred to as
Saccharomyces cerevisiae GRF18/pJDB207/GAPFL-HIR (Asp 63),
Saccharomyces cerevisiae GRF18/pJDB207/GAPFL-HIR (Glu 63),
Saccharomyces cerevisiae GRF18/pJDB207/GAPFL-HIR (Pro 66),
Saccharomyces cerevisiae HT246/pJDB207/GAPFL-HIR (Asp 63),
Saccharomyces cerevisiae HT246/pJDB207/GAPFL-HIR (Glu 63),
Saccharomyces cerevisiae HT246/pJDB207/GAPFL-HIR (Pro 66).

## Example 12: Recovery of desulphatohirudin mutants from S. cerevisiae

### a) Cultivation of the transformants

Cells of S. cerevisiae GRF18 und HT246 transformants (Example 11) are precultured twice by agitating in 10 ml of yeast minimal medium (Difco Yeast Nitrogen base without amino acids with the addition of 2 % glucose, 10 mg/l L-asparagine and 10 mg/l L-histidine) at 28°C and cultivated to a cell density of $3 \times 10^7$ cells/ml. These cells are then used for inoculating 50 ml cultures in full medium. The full medium is prepared with the composition: Difco Yeast Extract 10 g/l, Bacto-Peptone 5.0 g/l, glucose 20 g/l, D(+) saccharose 40 g/l, (NH₄)₂SO₄ 3.0 g/l, KH₂PO₄ 2.0 g/l, MgSO₄ 0.5 g/l, NaCl 0.1 g/l, and CaCl₂·2H₂O 0.1 g/l. The cultures are inoculated in full medium up to a cell density of $4 \times 10^6$ cells/ml and agitated at 28°C for 48 h at 250 revs/min.

b) Isolation of the desulphatohirudin mutants from the culture medium

Culture medium (1 l) is harvested after fermentation and centrifuged (1000 x g, 30 min) to separate cells from the supernatant. Acetonitrile 5 % (v/v) is added to the supernatant and the resulting solution is applied to a semi-preparative C18 RP-HPCL column at a rate of 24 ml/min. The column is washed with 90 % elution buffer A/10 % elution buffer B for 10 min and the gradient is started (conditions No. 1).

Fractions are collected from 30 to 65 min of the gradient. The fraction containing the hirudin mutant of interest, as identified by means of the thrombin inhibition assay [Griessbach, U. et al., Thromb. Res. 24, 221-224 (1984)], analytical FPLC analysis (conditions No. 2) and analytical HPLC analysis (conditions No. 3), is lyophilized.

The resulting solid is dissolved in H2O and filtered (0,2 μm) prior to separation by semi-preparative FPLC on a strong anion exchanger (Mono-Q) eluting with a pH gradient (conditions No. 2). The peak containing the majority of protein is connected as a single fraction and lyophilized twice from H2O.

In a final purification and desalting step, the lyophilized material is solubilized in water, injected onto a semi-preparative C18 RP-HPLC column and eluted (conditions No. 1). The pure desulphatohirudin mutant is collected manually as a single peak and lyophilized yielding a white solid.

Experimental conditions No. 1.

| | |
|---|---|
| Method: | Semi-preparative reversed phase high performance liquid chromatography (RP-HPLC) with gradient elution. |
| Column type: | LiChrosorb RP-18 (E. MERK, Darmstadt, FRG) |
| Particle size: | 10 μm |
| Dimensions: | 16 x 250 mm |
| Sample volume: | 1000 ml |
| Protein load/injection: | 5-50 mg |
| Flow rate: | 24 ml/min |
| Counter pressure: | 100-120 bar |
| Detection: | at 215 nm |
| Eluent A: | water (Analyzed HPLC Reagent, BAKER Chemicals, Deventer, Holland) with 0.1 % (v/v) trifluoroacetic acid (TFA) |
| Eluent B: | acetonitrile (for HPLC, Fluka Chemie AG, Buchs, Switzerland) with 0.07 % (v/v) TFA |

| Elution conditions: | time (min) | % B (v/v) |
|---|---|---|
| | 0 | 10 |
| | 10 | 10 |
| | 20 | 18 |
| | 30 | 18 |
| | 45 | 35 |
| | 65 | 35 |
| | 66 | 100 |
| | 70 | 100 |
| | 75 | 10 |

Experimental conditions No. 2.

| | |
|---|---|
| Method: | Fast protein liquid chromatography (FPLC) utilizing ion exchange chromatography on a strong anion exchanger stationary phase with pH-gradient elution. |
| Column type: | Mono-Q (Pharmacia, Uppsala, Sweden) |
| Dimensions: | 5 x 50 mm |
| Sample volume: | 4 ml (semi-preparative) |
| | 200 µl (analytical) |
| Protein load/injection: | 2-3 mg (semi-preparative) 500-600 µg/ml |
| | 25-200 µg (analytical) 125-1000 µg/ml |
| Flow rate: | 2.0 ml/min |
| Counter pressure: | 30-35 bar |
| Detection: | at 280 nm |
| Eluent A: | 50 mM $HCOONH_4$ pH = 4.5 |
| Eluent B: | 50 mM $HCOONH_4$ pH = 3.5 |

Elution conditions:

| time (min) | % B (v/v) |
|---|---|
| 0 | 0 |
| 5.0 | 0 |
| 19.5 | 100 |
| 22.0 | 100 |
| 22.1 | 0 |
| 25.0 | 0 |

17

Experimental conditions No. 3.

| | |
|---|---|
| Method: | Analytical reversed phase high performance liquid chromatography (RP-HPLC) with gradient elution. |
| Column type: | Nucleosil 100-5 $C_{18}$ (Macherey-Nagel, Düren, FRG) |
| Particle size: | 5 μm |
| Dimensions: | 4 x 120 mm |
| Sample volume: | 50 μl |
| Protein load/injection: | 2.5-10 μg (50-200 μg/ml) |
| Flow rate: | 1.5 ml/min |
| Counter pressure: | 120-150 bar |
| Detection: | at 215 nm |
| Eluent A: | water (Analyzed HPLC Reagent, BAKER Chemicals, Deventer, Holland) with 0.1 % (v/v) trifluoroacetic acid (TFA) |
| Eluent B: | acetonitrile (for HPLC, Fluka Chemie AG, Buchs, Switzerland) with 0.07 % (v/v) TFA |

Elution conditions:

| time (min) | % B (v/v) |
|---|---|
| 0 | 10 |
| 1 | 20 |
| 8 | 20 |
| 18 | 31 |
| 20 | 95 |
| 21.6 | 100 |
| 23 | 10 |

Example 13: Characterization of [Pro66]-desulphatohirudin and [Glu63]-desulphatohirudin

Samples of recombinant desulphatohirudin (cpd. 1), [Pro66]-desulphatohirudin (cpd. 2) and [Glu63]-desulphatohirudin (cpd. 3) are subjected to a thrombin inhibition test (Chromozyme-TH as chromogenic substrate; Griessbach, U. et al., Thromb. Res. 24, 221-224 (1984)) and to one or more analytical methods (see experimental conditions, example 12). The results are compiled in the following table for comparison.

| Experimental condition No. | Retention time (min) | | |
|---|---|---|---|
| | cpd. 1 | cpd. 2 | cpd. 3 |
| 2. FPLC | 16.0 | 14.7 | 17.5 |
| 3. RP-HPLC | 15.6 | 16.4 | 10.7 |
| thrombin inhibition (%) | ~ 100 | ~ 60 | ~ 35 |

a.) Determination of the amino acid composition

Approximately 2.5 μg of the pure desulphatohirudin are hydrolyzed at 110°C with the vapor of 6N HCl and are analyzed as described by Knecht and Chang [Anal. Chem. 58, 2375 (1986)].

The hydrolysates have the following composition:

[Pro66]-desulphatohirudin

| | | | | | | |
|---|---|---|---|---|---|---|
| Asx | 8.95 | (9) | Ile | 2.1 | (2) |
| Glx | 12.77 | (13) | Leu | 4.49 | (4) |
| Ser | 4.14 | (4) | Phe | 1.23 | (1) |
| Thr | 4.20 | (4) | Lys | 3.31 | (3) |
| Gly | 9.11 | (9) | His | 1.09 | (1) |
| Pro | 4.46 | (4) | Tyr | 2.29 | (2) |
| Val | 2.44 | (4) | Cys | 4.7 | (6) |
| | | | Total | | (66) |

The analysis is identical with recombinant desulphatohirudin [Grossenbacher, H. et al., Thromb. Res. Suppl. 7, 33 (1987)] except for the addition of one proline residue.

[Glu63]-desulphatohirudin

| | | | | | | |
|---|---|---|---|---|---|---|
| Asx | 8.79 | (9) | Ile | 1.95 | (2) |
| Glx | 13.31 | (14) | Leu | 4.26 | (4) |
| Ser | 3.86 | (4) | Phe | 1.17 | (1) |
| Thr | 4.19 | (4) | Lys | 3.36 | (3) |
| Gly | 8.42 | (9) | His | 1.32 | (1) |
| Pro | 3.11 | (3) | Tyr | 1.29 | (1) |
| Val | 2.73 | (4) | Cys | 5.82 | (6) |
| | | | Total | | (65) |

The analysis is identical with recombinant desulphatohirudin except for the addition of one glutamate residue and the absence of one tyrosine residue.

b.) Partial sequence analysis

To establish the N-terminal amino acid sequence, the pure desulphatohirudin mutants are subjected to conventional Edman degradation using an automated system and the N-terminal phenylthiohydantoin amino acids are separated by means of reverse phase HPLC.

Results:

[Pro66]-desulphatohirudin

Results:

[Pro⁶⁶]-desulphatohirudin

|  | 1 | 5 | 10 |
|---|---|---|---|
| Amino acid | Val-Val-Tyr-Thr-Asp-n.d.-Thr-Glu-Ser-Gly | | |

|  | 11 | 15 |
|---|---|---|
| Amino acid | Gln-Asn-Leu-n.d.-Leu-n.d. | |

[Glu⁶³]-desulphatohirudin

|  | 1 | 5 | 10 |
|---|---|---|---|
| Amino acid | Val-Val-Tyr-Thr-Asp-n.d.-Thr-Glu-Ser-Gly | | |

|  | 11 | 15 |
|---|---|---|
| Amino acid | Gln-Asn-Leu-n.d.-Leu-n.d. | |

n.d.= not determined

The N-terminal amino acid sequences of the desulphatohirudin mutants as shown are identical with that of authentic desulphatohirudin.

c.) C-terminal analysis
The pure desulphatohirudin mutants are digested with carboxypeptidase Y at room temperature. The released amino acids are derivatized with DABS-Cl and determined in an amino acid analyzer [Chang, Y.J. et al., Biochem. J. 203, 803-806 (1982)].

Results:

[Pro⁶⁶]-desulphatohirudin
C-terminal amino acids:
--Glu-Ile-Pro-Glu-Glu-[Tyr-Leu-Gln-Pro]

[Glu⁶³]-desulphatohirudin
C-terminal amino acids:
--Glu-Glu-[Ile-Pro]-Glu-Glu-Glu-[Leu-Gln]
The exact order of the amino acid sequence in brackets could not be determined.

d.) Molecular weight determination
Determination by fast atom bombardment positive ion mass spectrometry (FAB-MS). Instrument: ZAB-HF mass spectrometer (VG-Analytical Ltd., Manchester); matrix: thioglycerol; Xenon bombardment; ion energy 10 KeV.

Results:

[Pro⁶⁶]-desulphatohirudin

| empirical formula | $C_{292}H_{447}N_{80}O_{111}S_6$ |
|---|---|
| molecular weight (calc.): | 7060.64 |
| molecular weight (found) | 7064.2 |

Determination by $^{252}Cf$ plasma desorption time of flight spectrometry ($^{252}CfPD$ TOF-MS) (Sunquist, B. and Macfarlane, R.D. (1985) Mass Spectrom. Rev. 4, 421). Instrument: Bio-Ion (Nordic AG, Uppsala, Sweden); matrix: water; accelerating potential 10 KeV.

20

Results:

[Pro[66]]-desulphatohirudin

empirical formula     $C_{292}H_{447}N_{80}O_{111}S_6$
molecular weight     7060.64
(calc.):
molecular weight     7060.7
(found)

[Glu[63]]-desulphatohirudin

empirical formula     $C_{283}H_{438}N_{80}O_{111}S_6$
molecular weight     6929.46
(calc.):
molecular weight     6929.8
(found)

The desulphatohirudin mutants described in examples 3-9 have been characterized in an analogous manner.

## Example 14: Kinetic characterization

### A. Preparation and characterization of thrombin:

Thrombin is purified and characterized as described by S.R. Stone and J. Hofsteenge [Biochemistry 25, 4622-4628 (1986)]. The concentration of active thrombin is determined by active site titration with 4-methyl umbelliferyl p-guanidinobenzoate [Jameson, G.W., Roberts, D.V., Adams, R.W., Kyle, W.S.A, and Elmore, D.T., Biochem. J. 131, 101-117 (1973)].

### B. Enzyme assays:

The release of p-nitroaniline that results from the hydrolysis of the peptidyl p-nitroanilide substrates by thrombin is followed by measuring the increase in absorbance at 405 nm with a Shimadzu UV 240 spectrophotometer. The assays are performed in polystyrene cuvettes at 37°C in 0.05 M Tris-HCl buffer, pH 7.8, which contains 0.1 % polyethyleneglycol 6000, 0.1 M NaCl and p-nitroanilide substrate. The substrate D-Val-Leu-Arg-p-nitroanilide (S-2266; Kabi Vitrum) is used at a concentration of 300 µM in tight-binding titration experiments which are used to determine the concentration of the mutant hirudins. For such assays a thrombin concentration of 1.0 nM is used. The substrate D-Phe-pipecolyl-Arg-p-nitroanilide (S-2238; Kabi Vitrum) at a concentration of 100 µM and a thrombin concentration of 20 to 50 pM are used in slowing-binding inhibition experiments to determine the kinetic characteristics of the mutant hirudins (see below). Assays are started by the addition of thrombin. The amount of product formed is calculated using an adsorption coefficient of 9,920 $M^{-1}cm^{-1}$ for p-nitroaniline at 405 nm and the concentration of the substrate is determined spectrophotometrically at 342 nm using an absorption coefficient of 8,270 $M^{-1}cm^{-1}$ [Lottenberg, R. & Jackson C.M., Biochim. Biophys. Acta 742, 558-564 (1983)].

### C. Kinetic theory and data analysis

Tight-binding titration:

The inhibition of an enzyme in the presence of a substrate can be represented by the following mechanism (Scheme 1):

$$E + S \xrightleftharpoons{K_m} ES \xrightarrow{k_o} E + P$$

$$+$$
$$I$$

$$k_1 \Big\downarrow \Big\uparrow k_2$$

$$EI \qquad\qquad\qquad\qquad\qquad\qquad Scheme\ 1$$

The dissociation of the inhibitor ($K_I$) is equal to $k_2/k_1$. If the binding of the inhibitor to the enzyme causes a significant depletion in the concentration of the free inhibitor, the variation of the steady-state velocity ($v_s$) with the total inhibitor concentration ($I_t$) will be described by equation (1) [Morrison, J.F., Biochim. Biophys. Acta 185, 269-286 (1969); Henderson, P.J.F., Biochem. J. 127, 321-333 (1972)]:

$$v_s = \frac{v_o}{2E_t} [sqr((K_{I'} + I_t - E_t)^2 + 4K_{I'}E_t) - (K_{I'} + I_t - E_t)] \qquad (1)$$

where $v_o$ is the velocity observed in the absence of the inhibitor, $E_t$ is the total enzyme concentration, and $K_{I'}$ is the apparent inhibition constant. If the inhibitor and the substrate compete for the active site, $K_{I'}$ is related to the dissociation constant of the inhibitor ($K_I$) by equation (2):

$K_{I'} = K_I(1 - S/K_m)$ (2)

where S is the concentration of substrate and $K_m$ is its Michaelis constant. If the concentration of the enzyme or inhibitor is not accurately known, an additional factor can be incorporated into equation (1) to allow for this fact [Williams, J.W, and Morrison, J.F., Methods Enzymol. 63, 437-467 (1979)]. For the inhibition of thrombin by hirudin, the concentration of thrombin is known from active-site titration, but the concentration of hirudin is only known by weight and the data can be analysed according to equation (3):

$$v_s = \frac{v_o}{2E_t} [sqr((K_{I'} + xI_x - E_t)^2 + 4K_{I'}E_t) - (K_{I'} + xI_x - E_t)] \qquad (3)$$

where $I_x$ is the concentration of the inhibitor in terms of weight per volume and x is a factor which, when multiplied together with $I_x$, will yield the molar concentration of the inhibitor.

In tight-binding titration experiments, the dissociation constant for hirudin and its concentration is determined by holding the concentration of thrombin constant and varying the concentration of hirudin over a range which includes several concentrations that are lower than the concentration of thrombin and several concentrations that are higher. The total number of data points is usually between 7 and 10. The steady-state velocities obtained at each hirudin concentration is then fitted by weighted nonlinear regression to equation (3). For the regression, the observed values for the steady-state velocity are weighted according to the reciprocal of their value. This type of weighting has been empirically found to give the best results [Stone, S.R., and Hofsteenge, J., Biochemistry 25, 4622-4628 (1986)].

Slow, tight-binding inhibition:

If the rate of interaction of the inhibitor with the enzyme is slow so that the inhibited steady-state velocity is only slowly achieved, the progress curve of product formation for the mechanism of Scheme 1 will be described by equation (4) [Cha, S., Biochem. Pharmacol. 25, 2695-2702 (1976); Williams, J.W., Morrison, J.F., and Duggleby, R.G., Biochemistry 18, 2567-2573 (1979)]:

$$P = v_s \cdot t + \frac{(v_o - v_s)(1 - d)}{d \cdot k'} \log \left(\frac{1 - d \cdot \exp(-k' \cdot t)}{1 - d}\right) \qquad (4)$$

where P is the amount of product formed at time t, d is a function of $E_t$, $I_t$ and $K_{I'}$, and k' is a function of these parameters and the observed second-order association rate constant ($k_{1'}$) for the interaction between the inhibitor and enzyme (Cha et al., supra ). In order to determine the kinetic parameters for the mutant hirudins, progress-curve data obtained at at least six different hirudin concentrations are fitted to equation (4) by nonlinear regression. This analysis yields estimates for $K_{I'}$, $k_{1'}$ and the observed dissociation rate constant ($k_{2'}$). It has previously been shown that the value of $k_{1'}$ is independent of the binding of the substrate at the active site but that the observed values of $k_{2'}$ and $K_{I'}$ should be divided by $(1 + [S]/K_m)$ in order to obtain the true values ($k_2$ and $K_I$; S.R. Stone and J. Hofsteenge, Biochemistry 25, 4622-4628 (1986); Stone, S.R., Braun, P.J., and Hofsteenge, J., Biochemistry 26, 4617-4624 (1987)]. For these calculations, the previously determined value of 3.6 µM for the $K_m$ of D-Phe-pipecolyl-Arg-p-nitroanilide is used [Hofsteenge, J., Taguchi, H., and Stone, S.R., Biochem. J. 237, 243-251 (1986)]. Using these kinetic methods the following values for $K_I$ and $k_1$ are obtained:

| form of desulphatohirudin (=H) | $10^{-8} \cdot k_1$ $M^{-1} \cdot s^{-1}$ | $K_I$ (pM) |
|---|---|---|
| recombinant desulphatohirudin | $1.37 \pm 0.03$ | $0.231 \pm 0.006$ |
| $[Gln^{61,62}]-H$ | $0.45 \pm 0.01$ | $0.907 \pm 0.042$ |
| $[Ala^{53}]-H$ | n.d. | $0.810 \pm 0.160$ |
| $[Pro^{66}]-H$ | $1.09 \pm 0.13$ | $0.252 \pm 0.013$ |

n.d. = not determined

Example 15: Pharmaceutical composition containing a desulphatohirudin mutant for parenteral administration

A solution containing a desulphatohirudin mutant according to any one of the preceding Examples is dialysed against a 0.9 % NaCl solution. The concentration of the solution is then adjusted by diluting with the same NaCl solution to 0.2 mg/ml or 2 mg/ml. These solutions are sterilized by ultrafiltration (membranes with 0.22 μm pores).

The sterilized solutions can be used directly, for example for intravenous administration.

Deposit of microorganisms

The following microorganisms were deposited at the Deutsche Sammlung von Mikroorganismen (DSM), Mascheroder Weg 1b, D-3300 Braunschweig (FRG).

| microorganismus | deposition date | accession number |
|---|---|---|
| Saccharomyces cerevisiae GRF18 | March 4, 1986 | DSM 3665 |
| Saccharomyces cerevisiae HT246 | April 15, 1987 | DSM 4084 |

**Claims**

1. Mutants of desulphatohirudin having the amino acid sequence

```
                                            10
H-Val-Val-Tyr-Thr-Asp-Cys-Thr-Glu-Ser-Gly-

                                            20
  -Gln-Asn-Leu-Cys-Leu-Cys-Glu-Gly-Ser-Asn-

                                            30
  -Val-Cys-Gly-Gln-Gly-Asn-Lys-Cys-Ile-Leu-

                                            40
  -Gly-Ser-Asp-Gly-Glu-Lys-Asn-Gln-Cys-Val-

                                            50
  -Thr-Gly-Glu-Gly-Thr-Pro-Lys-Pro-Gln-Ser-

                                            60
  -His-Asn-Y₁-Gly-Asp-Phe-Y₂-Y₃-Ile-Pro-


  -Y₄-Y₅-Y₆-Leu-Y₇-OH
```

$$-His-Asn-Y_1-Gly-Asp-Phe-Y_2-Y_3-Ile-Pro-$$

$$-Y_4-Y_5-Y_6-Leu-Y_7-OH$$

(I)

wherein $Y_1$ represents Asp or the radical of a neutral genetically encoded amino acid, $Y_2$ and $Y_3$ independently from each other represent Glu, Asn or the radical of a lipophilic genetically encoded amino acid, $Y_4$ and $Y_5$ independently from each other represent Glu or the radical of a neutral genetically encoded amino acid, $Y_6$ represents Tyr or the radical of an acidic genetically encoded amino acid and $Y_7$ represents Gln or the dipeptidyl radical GlnPro, with the exception of compounds of the formula I in which $Y_1$ is Asp, $Y_6$ is Tyr, $Y_7$ is Gln, $Y_2$ and $Y_3$ are each Glu and one of the radicals $Y_4$ and $Y_5$ is Gln and the other is Glu, and salts thereof.

2. Desulphatohirudin mutants of the formula I according to claim 1 wherein $Y_1$ is Asp, Ala, Leu or Asn, $Y_2$ and $Y_3$ independently from each other represent Glu, Leu or Asn, $Y_4$ and $Y_5$ independently from each other represent Glu, Gln, Asn or Leu, $Y_6$ is Tyr, Asp or Glu and $Y_7$ is Gln or the dipeptidyl radical GlnPro, with the exception of compounds of the formula I in which $Y_1$ is Asp, $Y_6$ is Tyr, $Y_7$ is Gln, $Y_2$ and $Y_3$ are each Glu and one of the radicals $Y_4$ and $Y_5$ is Gln and the other is Glu, and salts thereof.

3. [Gln⁶¹,⁶²]-desulphatohirudin according to claim 1.

4. [Leu⁶¹,⁶²]-desulphatohirudin according to claim 1.

5. [Asn⁶¹,⁶²]-desulphatohirudin according to claim 1.

6. [Leu⁵⁷,⁵⁸,⁶¹,⁶²]-desulphatohirudin according to claim 1.

7. [Asn⁵⁷,⁵⁸,⁶¹,⁶²]-desulphatohirudin according to claim 1.

8. [Ala⁵³]-desulphatohirudin according to claim 1.

9. [Asp⁶³]-desulphatohirudin according to claim 1.

10. [Glu⁶³]-desulphatohirudin according to claim 1.

11. [Pro⁶⁶]-desulphatohirudin according to claim 1.

12. A pharmaceutical composition comprising a desulphatohirudin mutant according to claim 1.

13. A desulphatohirudin mutant according to claim 1 for use in a method for the prophylactic or therapeutic treatment of the human or animal body.

14. Method for the production of a desulphatohirudin mutant according to claim 1 comprising culturing a microbial host strain which has been transformed with a hybrid vector comprising an expression cassette consisting of a promoter operably linked to a first DNA sequence encoding a signal peptide linked in the proper reading frame to a second DNA sequence coding for the desulphatohirudin mutant, and a DNA sequence containing transcription termination signals, and isolating the desulphatohirudin mutant, and, if desired, converting an obtained polypeptide having free carboxy and/or amino groups into a salt or converting a salt obtained into the free compound.

15. A desulphatohirudin mutant obtainable by the method according to claim 14.

16. A hybrid vector comprising an expression cassette consisting of a promoter operably linked to a first DNA sequence encoding a signal peptide linked in the proper reading frame to a second DNA sequence coding for a desulphatohirudin mutant according to claim 1, and a DNA sequence containing transcription termination signals.

17. Method for the production of a hybrid vector according to claim 16 comprising linking the expression cassette consisting of a promoter operably linked to a first DNA sequence encoding a signal peptide linked in the proper reading frame to a second DNA sequence coding for the desulphatohirudin mutant, and a DNA sequence containing transcription termination signals, or the constituents of the expression cassette to the DNA fragments containing selective genetic markers and origins of replication for the selected microbial host in the predetermined order.

18. A microbial host strain which has been transformed with a hybrid vector comprising an expression cassette consisting of a yeast promoter operably linked to a first DNA sequence encoding a signal peptide linked in the proper reading frame to a second DNA sequence coding for the desulphatohirudin mutant according to claim 1, and a DNA sequence containing transcription termination signals.

19. Method for the production of the transformed microbial host strain according to claim 18 comprising transforming a microbial host strain with the hybrid vector according to claim 16.

**Claims for the following Contracting States: GR and ES**

1. A process for the production of mutants of desulphatohirudin having the amino acid sequence

$$10$$

$$H-Val-Val-Tyr-Thr-Asp-Cys-Thr-Glu-Ser-Gly-$$

$$20$$

$$-Gln-Asn-Leu-Cys-Leu-Cys-Glu-Gly-Ser-Asn-$$

$$30$$

$$-Val-Cys-Gly-Gln-Gly-Asn-Lys-Cys-Ile-Leu-$$

$$40$$

$$-Gly-Ser-Asp-Gly-Glu-Lys-Asn-Gln-Cys-Val-$$

$$50$$

$$-Thr-Gly-Glu-Gly-Thr-Pro-Lys-Pro-Gln-Ser-$$

$$60$$

$$-His-Asn-Y_1-Gly-Asp-Phe-Y_2-Y_3-Ile-Pro-$$

$$-Y_4-Y_5-Y_6-Leu-Y_7-OH$$

$$(I)$$

wherein $Y_1$ represents Asp or the radical of a neutral genetically encoded amino acid, $Y_2$ and $Y_3$ independently from each other represent Glu, Asn or the radical of a lipophilic genetically encoded amino acid, $Y_4$ and $Y_5$ independently from each other represent Glu or the radical of a neutral genetically encoded amino acid, $Y_6$ represents Tyr or the radical of an acidic genetically encoded amino acid and $Y_7$ represents Gln or the dipeptidyl radical GlnPro, with the exception of compounds of the formula I in which $Y_1$ is Asp, $Y_6$ is Tyr, $Y_7$ is Gln, $Y_2$ and $Y_3$ are each Glu and one of the radicals $Y_4$ and $Y_5$ is Gln and the other is Glu, and salts thereof, which process comprises culturing a microbial host strain which has been transformed with a hybrid vector comprising an expression cassette consisting of a promoter operably linked to a first DNA sequence encoding a signal peptide linked in the proper reading frame to a second DNA sequence coding for the desulphatohirudin mutant, and a DNA sequence containing transcription termination signals, and isolating the desulphatohirudin mutant, and, if desired, converting an obtained polypeptide having free carboxy and/or amino groups into a salt or converting a salt obtained into the free compound.

2. A process for the production of desulphatohirudin mutants of the formula I according to claim 1 wherein $Y_1$ is Asp, Ala, Leu or Asn, $Y_2$ and $Y_3$ independently from each other represent Glu, Leu or Asn, $Y_4$ and $Y_5$ independently from each other represent Glu, Gln, Asn or Leu, $Y_6$ is Tyr, Asp or Glu and $Y_7$ is Gln or the dipeptidyl radical GlnPro, with the exception of compounds of the formula I in which $Y_1$ is Asp, $Y_6$ is Tyr, $Y_7$ is Gln, $Y_2$ and $Y_3$ are each Glu and one of the radicals $Y_4$ and $Y_5$ is Gln and the other is Glu, and salts thereof.

3. A process for the production of [Gln$^{61,62}$]-desulphatohirudin according to claim 1.

4. A process for the production of [Leu$^{61,62}$]-desulphatohirudin according to claim 1.

5. A process for the production of [Asn$^{61,62}$]-desulphatohirudin according to claim 1.

6. A process for the production of [Leu$^{57,58,61,62}$]-desulphatohirudin according to claim 1.

7. A process for the production of [Asn$^{57,58,61,62}$]-desulphatohirudin according to claim 1.

8. A process for the production of [Ala$^{53}$]-desulphatohirudin according to claim 1.

9. A process for the production of [Asp$^{63}$]-desulphatohirudin according to claim 1.

10. A process for the production of [Glu$^{63}$]-desulphatohirudin according to claim 1.

11. A process for the production of [Pro$^{66}$]-desulphatohirudin according to claim 1.

12. A process for the production of a pharmaceutical composition which process comprises admixing a desulphatohirudin mutant of the formula I that can be produced in accordance with claim 1, or a pharmaceutically acceptable salt thereof, to a pharmaceutically acceptable carrier and/or adjuncts.

13. A process for the production of a hybrid vector comprising an expression cassette consisting of a promoter operably linked to a first DNA sequence encoding a signal peptide linked in the proper reading frame to a second DNA sequence coding for the desulphatohirudin mutant that can be produced according to claim 1, and a DNA sequence containing transcription termination signals, which process comprises linking the expression cassette consisting of a promoter operably linked to a first DNA sequence encoding a signal peptide linked in the proper reading frame to a second DNA sequence coding for the desulphatohirudin mutant, and a DNA sequence containing transcription termination signals, or the constituents of the expression cassette to the DNA fragments containing selective genetic markers and origins of replication for the selected microbial host in the predetermined order.

14. A process for the production of a microbial host strain which has been transformed with a hybrid vector comprising an expression cassette consisting of a yeast promoter operably linked to a first DNA sequence encoding a signal peptide linked in the proper reading frame to a second DNA sequence coding for the desulphatohirudin mutant that can be produced according to claim 1, and a DNA sequence containing transcription termination signals, which process comprises transforming a microbial host strain with said hybrid vector.

15. A microbial host strain obtainable by the process according to claim 14.

26

## *Fig. 1:* Construction of plasmid pML350

```
······GTA GCG CAG GCC  GCT GAA TTC CAA GCT TGG ATC CGG GTG
······Val Ala Gln Ala  Ala Glu Phe Gln Ala Trp Ile Arg ...
```

**omp A signal peptide**

```
······GTA GCG CAG GCC  GCT GAA TTC ATG GTT GTT······
······Val Ala Gln Ala  Ala Glu Phe Met Val Val ······
```

**omp A signal peptide**　　　　　**hirudin gene**

**_Fig. 2:_ Plasmid map of plasmid pJDB207/GAPFL-HIR**

**_Fig. 3:_ Construction of plasmid pJDB207/GAPFL-HIR (Asp 63)**

pJDB207/GAPFL-HIR

1. Sal I digestion
2. Eco RI digestion
3. isolation of 478 bp fragment

isolation of 740 bp fragment

1. Bam HI digestion
2. Sal I digestion

isolation of 6.7 kb fragment

GAPFL p

SalI [Bam/Bgl] EcoRI

SalI [Bam/Bgl] AsuII EcoRI AsuII BamHI

SalI

1. Asu II digestion
2. linker (L) ligation
3. Bam HI digestion
4. Eco RI digestion
5. isolation of 262 bp fragment

HIR L

EcoRI AsuII BamHI

Bam HI

EcoRI

EcoRI

ligation, transformation

pJDB207-GAPFL-HIR

7494 SalI
AsuII
EcoRI
AsuII
BamHI
GAPFL-HIR
7000
HIR
1000
HindIII
6000
pAT153
IR2
XbaI
ORI
2MICRON
2000
AMP
REP3
ScaI
5000
dLEU2
HpaI
2MI
EcoRI
AsuII
3000
BstEII
4000
AsuII
EcoRI ClaI

pJDB207/GAPFL-HIR (Asp 63)